# EUROPEAN PATENT APPLICATION

(11) **EP 2 356 990 A2**
(43) Date of publication of application: **17.08.2011**
(21) Application number: 10187259.6
(22) Date of filing: 02.08.2007
(51) Int. Cl.: A61K 31/70, A61P 31/14, A61P 31/18, A61P 31/20, A61K 9/127, A61K 38/02, A61K 38/08

(54) **Liposome treatment of viral infections**

(30) Priority: 02.08.2006 US 834797 P; 22.09.2006 US 846344 P
(62) Divisional of application: 07872266.7
(71) Applicant: University of Oxford, Oxford OX1 3QU (GB)
(72) Inventor: Dwek, Raymond.A., Oxford OX1 3QU (GB); Nichita-Branza, Norica, 77700 Bucharest (RO); Petrescu, Stefana, 77700 Bucharest (RO); Pollock, Stéphanie, Oxford OX2 6PX (GB); Rudd, Pauline, Abingdon,Oxford. OX1 42PA (GB); Scanlan, Christopher, Oxford OX3 8FF (GB); Zitzmann, Nicole, Oxford OX1 4TL (GB)
(74) Representative: Warcoin, Jacques

(57) **Abstract**

One can treat a viral infection such as hepatitis B (HBV), hepatitis C (HCV), and bovine viral diarrhea virus (BVDV) infections via the delivery of pH sensitive liposomes directly into the endoplasmic reticulum (ER) membrane. Two exemplary liposome formulations are DOPE/CHEMS (DC liposomes) and DOPE/CHEMS/PEG-PE (DCPP liposomes). DC and DCPP liposomes can optimize the intracellular delivery of *N*-butyl deoxynojirimycin (NB-DNJ), and consequently increase the *in* vivo activity of this iminosugar several orders of magnitude, and could be used in combination with other therapeutic agents such as interferon and/or ribavirin. The optimized release of *N*B-DNJ directly into the ER can be also applied for the treatment of other viruses, for which NB-DNJ is known to be an effective antiviral, such as human immunodeficiency virus (HIV).

SEQUENCE LISTING

<110>  UNIVERSITY OF OXFORD

<120>  LIPOSOME TREATMENT OF VIRAL INFECTIONS

<130>  68905D27294

<150>  US60/834,797
<151>  2006-08-02

<150>  US60/846,344
<151>  2006-09-22

<160>  2

<170>  PatentIn version 3.3

<210>  1
<211>  20
<212>  DNA
<213>  artificial

<220>
<223>  Forward primer

<400>  1
tagggcaaac catctggaag                                                    20


<210>  2
<211>  20
<212>  DNA
<213>  artificial

<220>
<223>  Reverse primer

<400>  2
acttggagct acaggcctca                                                    20

## Description

### RELATED APPLICATIONS

The present application claims priority to US provisional applications Nos. 60/834,797 filed August 2, 2006, to Dwek et al. and 60/846,344 filed September 22, 2006, to Dwek et al., which are both incorporated herein by reference in their entirety.

### FIELD

The present application relates generally to methods and compositions for treatment of viral infections and, more specifically, to methods and compositions for treatment of viral infections utilizing liposomes.

### BACKGROUND

*Hepatitis B virus.* Hepatitis B virus (HBV, HepB) is a causative agent of acute and chronic liver disease including liver fibrosis, cirrhosis, inflammatory liver disease, and hepatic cancer that can lead to death in some patients, see e.g. Joklik, Wolfgang K., Virology, Third Edition, Appleton & Lange, Norwalk, Conn., 1988 (ISBN 0-8385-9462-X). Although effective vaccines are available, more than 280 million people worldwide, i.e. 5 % of the world's population, are still chronically infected with the virus, see e.g. Locamini, S. A., et. al., Antiviral Chemistry & Chemotherapy (1996) 7(2):53-64. Such vaccines have no therapeutic value for those already infected with the virus. In Europe and North America, between 0.1 % and 1 % of the population is infected. Estimates are that 15 % to 20 % of individuals who acquire the infection develop cirrhosis or another chronic disability from HBV infection. Once liver cirrhosis is established, morbidity and mortality are substantial, with about a 5 year patient survival period, see e.g. Blume, H., E., et.al., Advanced Drug Delivery Reviews (1995) 17:321-331.

*Hepatitis C virus.* Approximately 170 million people worldwide, i.e. 3 % of the world's population, see e.g. WHO, J. Viral. Hepat. 1999; 6: 35-47, and approximately 4 million people in the United States are infected with Hepatitis C virus (HCV, HepC). About 80 % of individuals acutely infected with HCV become chronically infected. Hence, HCV is a major cause of chronic hepatitis. Once chronically infected, the virus is almost never cleared without treatment. In rare cases, HCV infection causes clinically acute disease and even liver failure. Chronic HCV infection can vary dramatically between individuals, where some will have clinically insignificant or minimal liver disease and never develop complications and others will have clinically apparent chronic hepatitis and may go on to develop cirrhosis. About 20 % of individuals with HCV who do develop cirrhosis will develop end-stage liver disease and have an increased risk of developing primary liver cancer.

Antiviral drugs such as interferon, alone or in combination with ribavirin, are effective in up to 80 % of patients (Di Bisceglie, A. M, and Hoofnagle, J. H. 2002, Hepatology 36, S121-S127), but many patients do not tolerate this form of combination therapy.

*Bovine viral diarrhea virus.* Bovine viral diarrhea virus (BVDV) is distributed worldwide and is prevalent in most cattle populations. BVDV is also commonly used as tissue culture surrogate of HCV. There are two viral biotypes of BVDV: noncytopathic (ncp) and cytopathic (cp). Classification of viral biotype is based on cytopathic effect in cultured cells and is not related to virulence. Ncp BVDV is common in cattle, while the cp biotype is relatively rare and arises from the ncp strain after a specific mutational event occurs in the viral genome. Infection of cells with the cp BVDV strain in tissue culture is characterized by formation of clusters of apoptotic cells (plaques) on the cell monolayer, which can be easily monitored microscopically.

*Human immunodeficiency virus.* Human immunodeficiency virus (HIV) is the causative agent of acquired immune deficiency syndrome (AIDS) and related disorders. There are at least two distinct types of HIV: HIV-1 and HIV-2. Further, a large amount of genetic heterogeneity exists within populations of each of these types. Since the onset of the AIDS epidemic, some 20 million people have died and the estimate is that over 40 million are now living with HIV-1/AIDS, with 14 000 people infected daily worldwide.

Numerous antiviral therapeutic agents and diagnostic capabilities have been developed that, at least for those with access, have greatly improved both the quantity and quality of life. Most of these drugs interfere with viral proteins or processes such as reverse transcription and protease activity. Unfortunately, these treatments do not eliminate infection, the unwanted effects of many therapies are severe, and drug resistant strains of HIV exist for every type of antiviral currently in use.

*N-butyldeoxynojirimycin(NB-DNJ) as a therapeutic agent.* NB-DNJ, also known as N-butyl-1,5-dideoxy-1,5-imino-D-glucitol, inhibits processing by the ER glucosidases I and II, and has been shown to be an effective antiviral by causing the misfolding and/or ER-retention of glycoproteins of human immunodeficiency virus (HIV) and hepatitis viruses, such as Hepatitis B virus, Hepatitis C virus, Bovine viral diarrhea, virus amongst others. Methods of synthesizing NB-DNJ and other N-substituted deoxynojirimycin derivatives are described, for example, in US parents Nos. 5,622,972, 4,246,345, 4,266,025, 4,405,714 and 4,806,650. Antiviral effects of NB-DNJ are discussed, for example, in US patents Nos. 6,465,487; 6,545,021; 6,689,759; 6,809,083 for hepatitis viruses and US patent No. 4,849,430 for HIV virus.

Glucosidase inhibitors, such as NB-DNJ, have been shown to be effective in the treatment of HBV infection in both cell culture and using a woodchuck animal model, see e.g. T. Block, X. Lu, A.S. Mehta, B.S. Blumberg, B. Tennant, M. Ebling, B. Korba, D.M. Lansky, G.S. Jacob & R.A. Dwek , Nat Med. 1998 May;4(5):610-4. NB-DNJ suppresses secretion of HBV particles and causes intracellular retention of HBV DNA.

NB-DNJ has been shown to be a strong antiviral against BVDV, a cell culture model for HCV, see e.g. Branza-Nichita N, Durantel D, Carrouee-Durantel S, Dwek RA, Zitzmann N., J Virol. 2001 Apr;75(8):3527-36; Durantel, D., et al, J. Virol., 2001, 75, 8987-8998; N. Zitzmann, et al , PNAS, 1999, 96, 11878-11882. Treatment with NB-DNJ leads to decreased infectivity of viral progeny, with less of an effect on the actual number of secreted viruses.

NB-DNJ has been shown to be antiviral against HIV; treatment leads to a relatively small effect on the number of virus particles released from HIV-infected cells, however, the amount of infectious virus released is greatly reduced, see e.g. P.B. Fischer, M. Collin, et al (1995), J. Virol. 69(9) :5791-7; P.B. Fischer, G.B. Karlsson, T. Butters, R. Dwek and F. Platt , J. Virol. 70 (1996a), pp. 7143-7152, P.B. Fischer, G.B. Karlsson, R. Dwek and F. Platt ,. J. Virol. 70 (1996b), pp. 7153-7160. Clinical trials involving NB-DNJ were conducted in HIV-1 infected patients, and results demonstrated that concentrations necessary for antiviral activity were too high and resulted in serious side-effects in patients, see e.g. Fischl M.A., Resnick L., Coombs R., Kremer A.B., Pottage J.C. Jr, Fass R.J., Fife K.H., Powderly W.G., Collier A.C., Aspinall R.L., et. al., J. Acquir. Immune. Defic. Syndr. 1994 Feb;.7(2):139-47. No mutant HIV strain resistant to NB-DNJ treatment currently exists.

*ER protein folding & glucosidases I αnd II.* The antiviral effect demonstrated by glucosidase inhibition is thought to be a result of misfolding or retention of viral glycoproteins within the ER, primarily through blocking entry into the calnexin/calreticulin cycle. Following transfer of the triglucosylated oligosaccharide (Glc₃Man₉GlcNAc₂) to an Asn-X-Ser/Thr consensus sequence in the growing polypeptide chain, it is necessary that the three α-linked glucose residues be released before further processing to the mature carbohydrate units can take place. Moreover, the two outer glucose residues must be trimmed to allow entry into the calnexin/calreticulin cycle for proper folding, see e.g. Bergeron, J.J. et. al., Trends Biochem. Sci., 1994, 19, 124-128; Peterson, J. R. et. al., Mol. Biol. Cell, 1995, 6, 1173-1184. The initial processing is affected by an ER-situated integral membrane enzyme with a lumenally-oriented catalytic domain (glucosidase I) that specifically cleaves the α1-2 linked glucose residue; this is followed by the action of glucosidase II, which releases both of the α1-3 linked glucose components.

*Liposomes.* Liposomes can deliver water-soluble compounds directly inside the cell, bypassing cellular membranes that act as molecular barriers. The pH sensitive liposome formulation can involve the combination of phopsphatidylethanolamine (PE), or its derivatives (e.g. DOPE), with compounds containing an acidic group, which can act as a stabilizer at neutral pH. Cholesteryl hemisuccinate (CHEMS) can be a good stabilizing molecule as its cholesterol group confers higher stability to the PE-containing vesicles compared to other amphiphilic stabilizers in vivo. The in vivo efficacy of liposome-mediated delivery can depend strongly on interactions with serum components (opsonins) that can influence their pharmacokinetics and biodistribution. pH-sensitive liposomes can be rapidly cleared from blood circulation, accumulating in the liver and spleen, however inclusion of lipids with covalently attached polyethylene glycol (PEG) can overcome clearance by the reticuloendothelial system (RES) by stabilizing the net-negative charge on DOPE:CHEMS liposomes, leading to long circulation times. DOPE-CHEMS and DOPE-CHEMS-PEG-PE liposomes and methods of their preparation are described, for example, in V.A. Slepushkin, S. Simoes, P. Dazin, M.S. Newman, L.S. Guo and M.C.P. de Lima, J. Biol. Chem. 272 (1997) 2382-2388; and S. Simoes, V. Slepushkin, N. Duzgunes and M.C. Pedroso de Lima, Biomembranes 1515 (2001) 23-37, both incorporated herein by reference in their entirety.

Delivery of NB-DNJ encapsulated in DOPE-CHEMS (molar ratio 6:4) is disclosed in US patent application No. US2003/0124160.

### SUMMARY

One embodiment provides a method of treating a viral infection, comprising administering to a host in need thereof a composition comprising (a) a liposome comprising DOPE and CHEMS lipids and (b) one or more therapeutic agents encapsulated into the liposome, wherein the viral infection is an ER membrane budding viral infection or a plasma membrane budding viral infection; wherein the one or more therapeutic agents comprise *N*-butyl deoxynojirimycin (*N*B-DNJ) and wherein said administering results in delivering of the one or more therapeutic agents into an endoplasmic reticulum of a cell, that is infected with a virus causing the infection, and incorporating one or more lipids of the liposome in an endoplasmic reticulum membrane of the cell.

Another embodiment of the invention provides a method of treating a viral infection, comprising administering to a host in need thereof a composition comprising (a) a liposome comprising DOPE, CHEMS and PEG-PE lipids and (b) one or more therapeutic agents encapsulated into the liposome. The one or more therapeutic agents can comprise *N-*butyl deoxynojirimycin (*N*B-DNJ).

Yet another embodiment provides a method comprising administering to a host in need thereof a composition comprising (a) a pH sensitive liposome and (b) an antigen encapsulated inside the liposome, wherein the administering results in increasing antigen presentation by a major histocompatibility molecule class 1 of a antigen presenting cell.

Yet another embodiment is a method of treating an HIV infection comprising administering to a host in need thereof a composition comprising a liposome conjugated with a gp120/gp41 complex targeting moiety.

And yet another embodiment is a composition comprising a liposome comprising DOPE, CHEMS and PEG-PE lipids and at least one therapeutic agent, such as *N*-butyl deoxynojirimycin (*N*B-DNJ) encapsulated inside the liposome.

And yet another embodiment is a composition, comprising a pH sensitive liposome and an antigen encapsulated inside the liposome.

And yet another embodiment is a composition comprising a liposome conjugated with a gp120/gp41 complex targeting moiety.

And yet according to another embodiment, a method of treating or preventing a viral infection, comprises administering to a host in need thereof a composition comprises (a) a liposome comprising PI lipids and (b) at least one antiviral therapeutic agent encapsulated into the liposome, wherein said contacting results in delivering of the at least one therapeutic agent into the ER lumen of a cell, that is infected with a virus causing the infection, and incorporating one or more lipids of the liposome in the ER membrane of the cell.

And yet according to another embodiment, a composition comprises a liposome comprising PI lipids and at least one antiviral therapeutic agent encapsulated inside the liposome.

And yet another embodiment is a method of treating a viral infection comprising administering to a host in need thereof a composition comprising (a) a liposome comprising PI lipids and (b) at least one antiviral protein intercalated into a lipid layer of the liposome, wherein said contacting results in incorporating one or more lipids of the liposome in an endoplasmic reticulum membrane of a cell, that is infected with a virus causing the infection.

And yet another embodiment is a composition comprising (a) a liposome comprising PI lipids and (b) at least one antiviral protein intercalated into a lipid layer of the liposome.

And yet another embodiment is a composition comprising a liposome comprising PI lipids and at least one therapeutic agent encapsulated inside the liposome.

And yet another embodiment is a method of treating or preventing a physiological condition comprising administering to a subject in need thereof a composition comprising a liposome comprising PI lipids and at least one therapeutic agent encapsulated inside the liposome.

And yet another embodiment is a composition comprising a liposome comprising PI lipids and at least one protein intercalated into a lipid bilayer of the liposome.

And yet another embodiment is a method of treating or preventing a physiological condition comprising administering to a subject in need thereof a composition comprising a liposome comprising PI lipids and at least one protein intercalated into a lipid bilayer of the liposome.

### BRIEF DESCRIPTION OF THE DRAWINGS

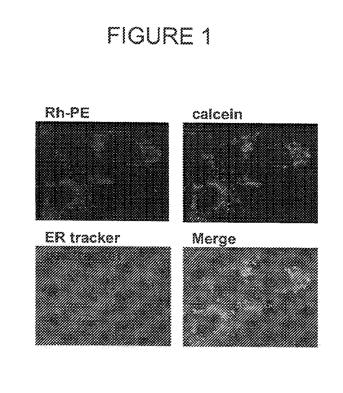

FIG. 1 demonstrates endoplasmic reticulum (ER) localization of dequenched calcein and fluorescence labelled lipids (Rh-PE) following delivery via DCPP-Rh liposomes.

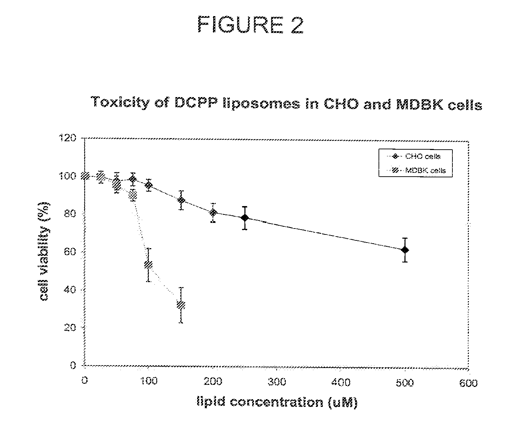

FIG. 2 shows toxicity of DCPP liposomes in CHO and MDBK cells. DCPP liposomes encapsulating PBS were added to CHO cells with final lipid concentrations ranging between 0-500 µM, and to MDBK cells with concentrations ranging between 0-150 µM. Cells and liposomes were left to incubate 5 days before cell viability was measured by trypan blue staining. Results are presented as the percentage of viable cells compared to the untreated control.

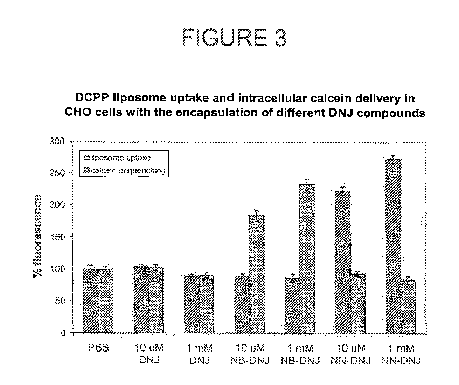

FIG. 3 is a plot showing DCPP-Rh liposome uptake and intracellular calcein dequenching in CHO cells with the encapsulation of deoxynojirimycin (DNJ), *N*-butyl deoxynojirimycin (*N*B-DNJ) and *N-*nonyl deoxynojirimycin (*N*N-DNJ) compounds. DCPP-Rh liposomes were prepared encapsulating each compound at two different concentrations. Liposome uptake, measured by incorporation of Rh-PE in cellular membranes, and calcein dequenching, a measure of intracellular release, was determined following a 5-min pulse with liposomes and a 30-min chase in a fresh medium.

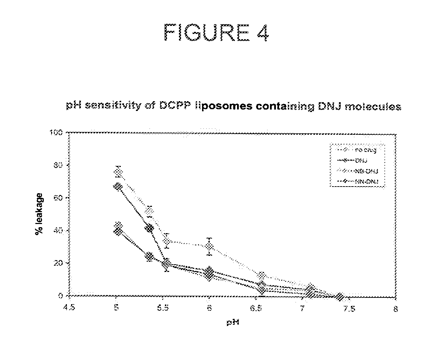

FIG. 4 shows pH sensitivity of DCPP liposomes containing various DNJ molecules.

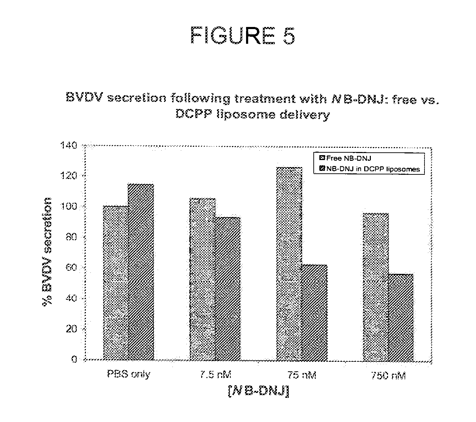

FIG. 5 is a plot showing BVDV secretion following treatment with *N*B-DNJ: free vs. DCPP liposome-mediated delivery. The secretion of BVDV particles from infected MDBK cells during treatment with *N*B-DNJ added either freely into the medium or via liposomes with a final lipid concentration of 50 µM was determined by real-time PCR following a 3 day incubation. Results are presented as a percentage of RNA copies detected by real-time PCR compared to the untreated control.

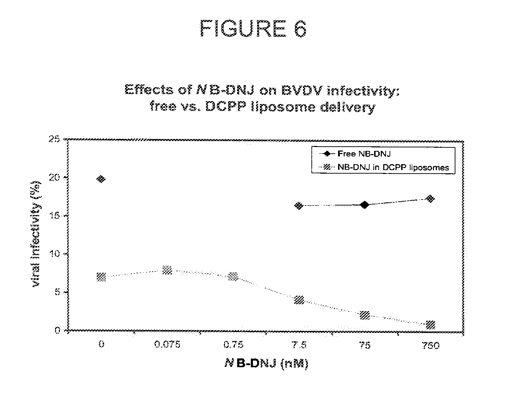

FIG. 6 demonstrates the effects of *N*B-DNJ on ncp BVDV infectivity: free vs. DCPP liposome-mediated delivery. Infectivity of ncp BVDV particles produced by infected MDBK cells in the presence of *N*B-DNJ, either added freely in the medium or via liposomes with a final lipid concentration of 50 µM, was measured by incubation with naive MDBK cells for 3 days. Infected cells were detected by immunofluorescent staining of non-structural BVDV proteins present in MDBK cells using DAPI counterstain as a control. Data from BVDV secretion (Figure 2) were used to normalize calculations for final percent infectivity.

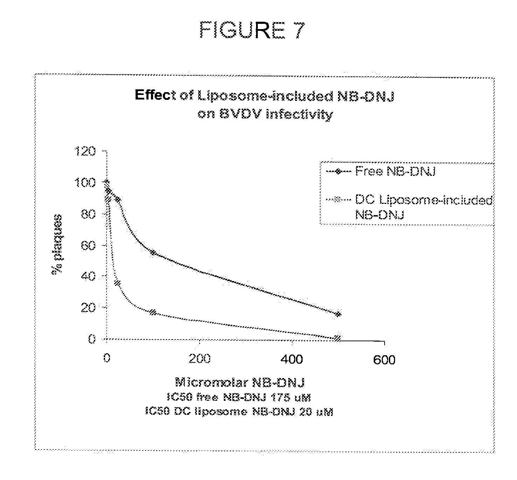

FIG. 7 shows the antiviral effect of *N*B-DNJ against cp BVDV: free vs. DC liposome-mediated delivery. MDBK cells infected with cp BVDV were grown in the presence of free or DC liposome-included NB-DNJ, for 3 days. The supernatants containing secreted virus were used to infect naive MDBK. After 3 days the resulting plaques were counted under the microscope (yield assay).

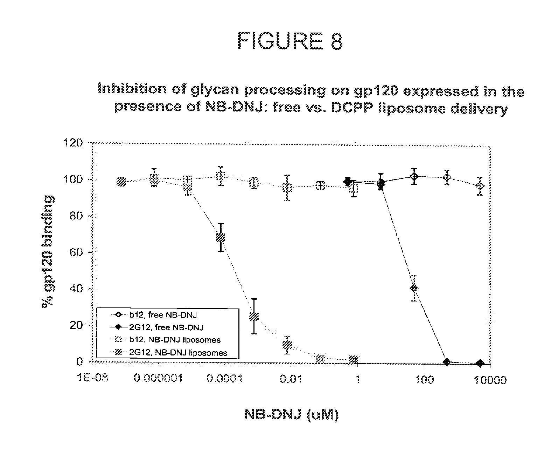

FIG. 8 demonstrates inhibition of glycan processing on the HIV envelope protein gp120 expressed in the presence of *N*B-DNJ: free vs. DCPP liposome-mediated delivery. CHO cells expressing a soluble form of gp120 were incubated with *N*B-DNJ, either added freely into the medium or via liposomes with a final lipid concentration of 100 µM. *N*B-DNJ activity, determined by the inhibition of glucose trimming by ER glucosidases, was measured by the binding of monoclonal antibodies 2G12 and b12 to the *N*B-DNJ-treated gp120 in capture ELISAs.

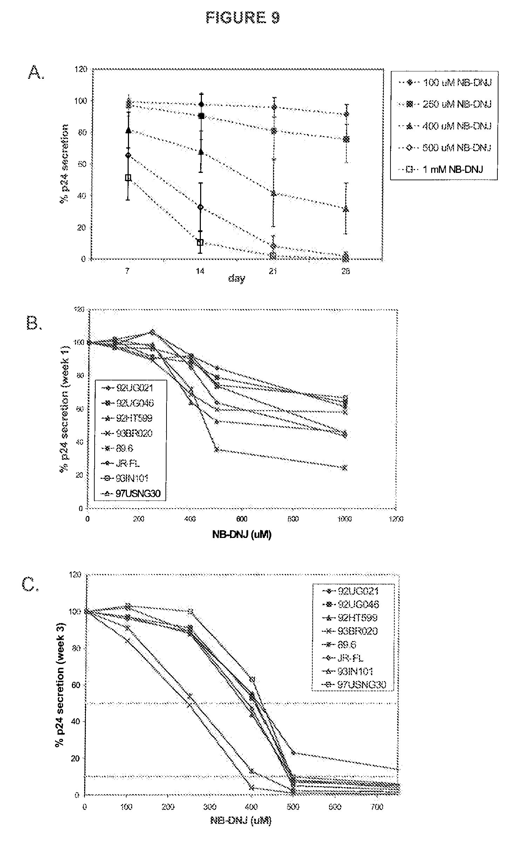

FIG. 9 (A-C) presents antiviral effects of free *N*B-DNJ on eight separate HIV-1 primary isolates. FIG. 9A shows average p24 secretion of the eight isolates treated with varying concentrations of *N*B-DNJ over four weeks. Error bars show the standard deviation between isolates for each treatment. FIG. 9B shows secretion of each primary isolate following initial treatment with free *N*B-DNJ at concentrations ranging 0 - 1 mM. FIG. 9C shows sensitivity of individual primary isolates to *N*B-DNJ following three weeks of treatment. All values are expressed as a percentage of the untreated control, and data represent the mean obtained from triplicate samples of two independent experiments. The approximate IC50s and IC90s for each treatment are indicated by grey (dotted) lines across the graph.

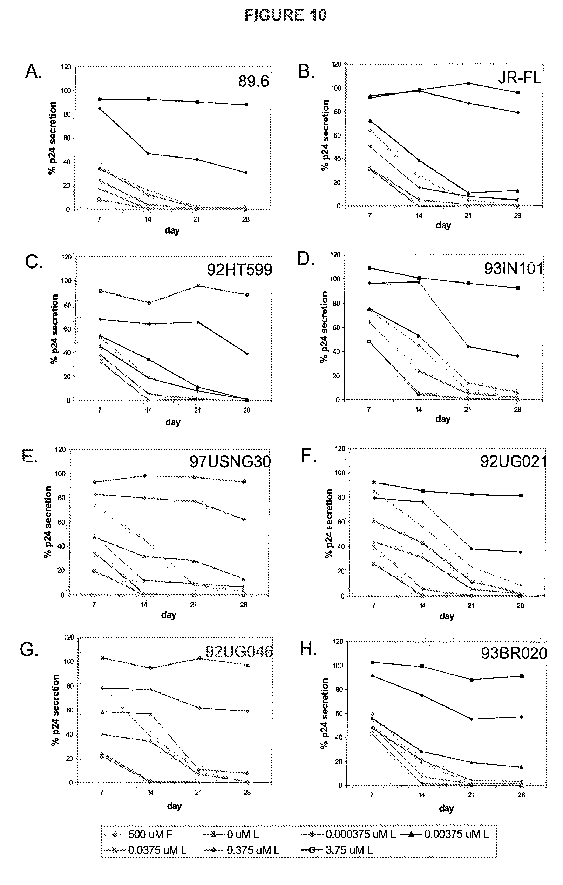

FIG. 10 A-H represent a data demonstrating how liposomes increase the antiviral activity of *N*B-DNJ against eight primary isolates of HIV-1. PBMCs infected with each isolate (represented in graphs A to H) were treated with liposomes (L) encapsulating *N*B-DNJ at various concentrations over a four week period. Treatment with 500 µM *N*B-DNJ free (F) in the media is shown as a reference for antiviral activity. The legend indicates the final concentration of *N*B-DNJ for each treatment. All values are expressed as a percentage of the untreated control, and data represent the mean obtained from triplicate samples of two independent experiments.

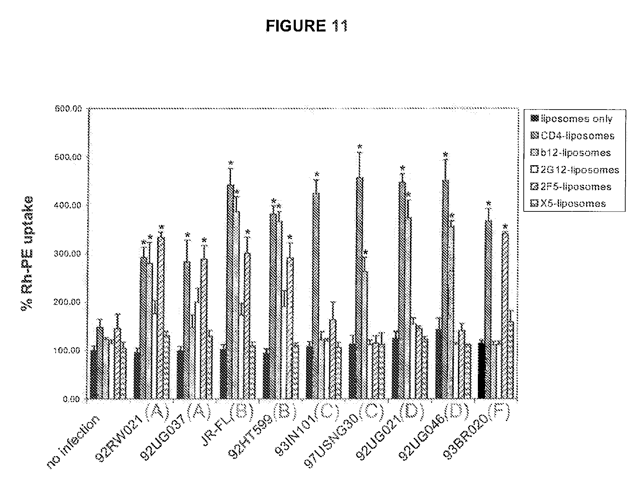

FIG. 11 shows uptake of sCD4-liposomes and immunoliposomes by cells infected with a broad range of HIV-1 isolates. sCD4- and MAb-liposome conjugates are incubated with PBMCs infected with nine different primary isolates (clades in brackets). Increased uptake is measured by the increase in fluorescent lipids in cells following incubation. All values were normalized to the 'no infection, liposome only' control and data represent the mean ± standard error obtained from triplicates of one experiment. The relative uptake data were tested separately for each infection using a series of one-way analyses of variance followed by post-hoc Tukey tests to test for differences between the effectiveness of different targeting molecules. Significant differences in uptake between liposome conjugates and the liposome only control (P<0.0001) are denoted with asterisks.

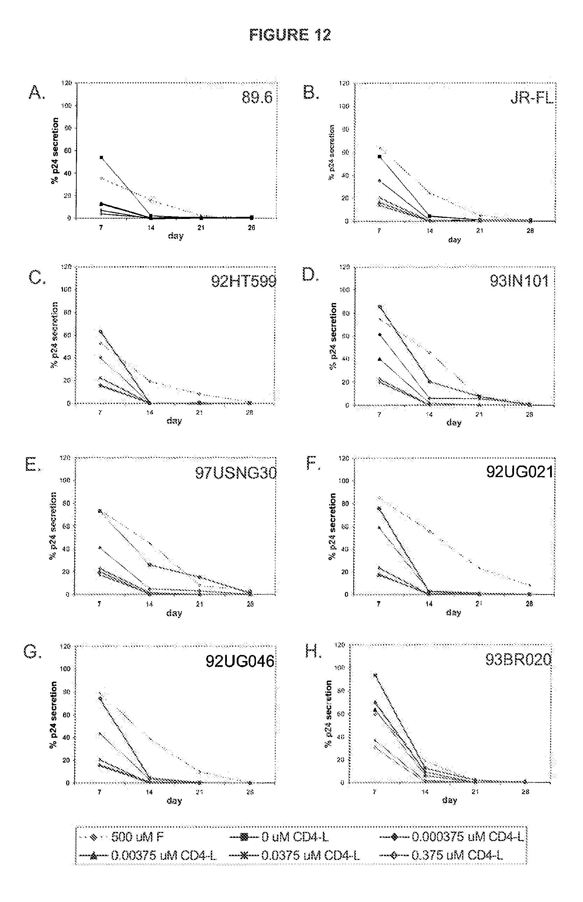

FIG. 12 A-H demonstrate potent synergistic antiviral activity of sCD4-liposomes encapsulating *N*B-DNJ. PBMCs infected with each isolate (represented in graphs A to H) were treated with sCD4-liposomes (CD4-L) encapsulating *N*B-DNJ at various concentrations over a four week period. Treatment with 500 µM *N*B-DNJ free (F) in the media is shown as a reference for antiviral activity. The legend indicates the final concentration of *N*B-DNJ for each treatment. All values are expressed as a percentage of the untreated control, and data represent the mean obtained from triplicate samples of one experiment.

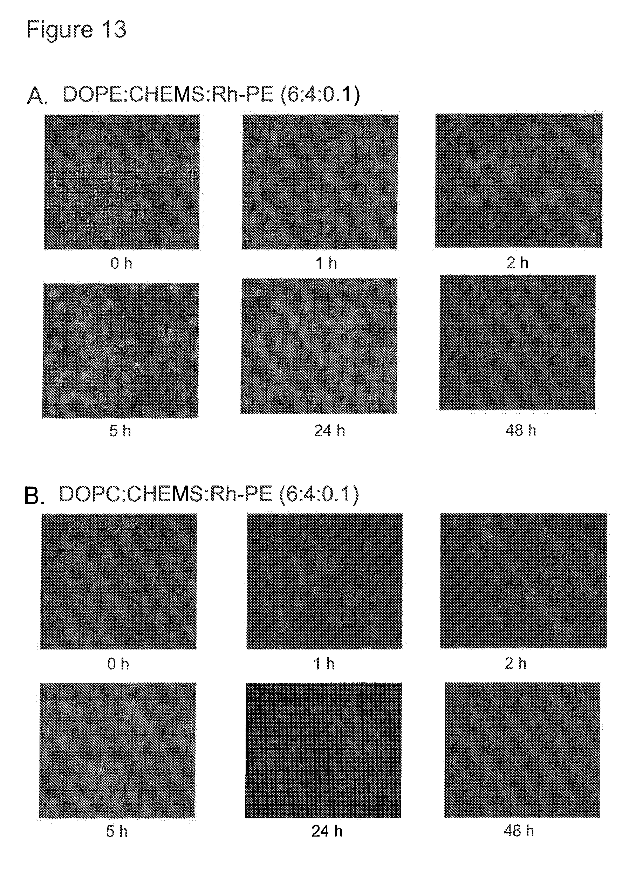

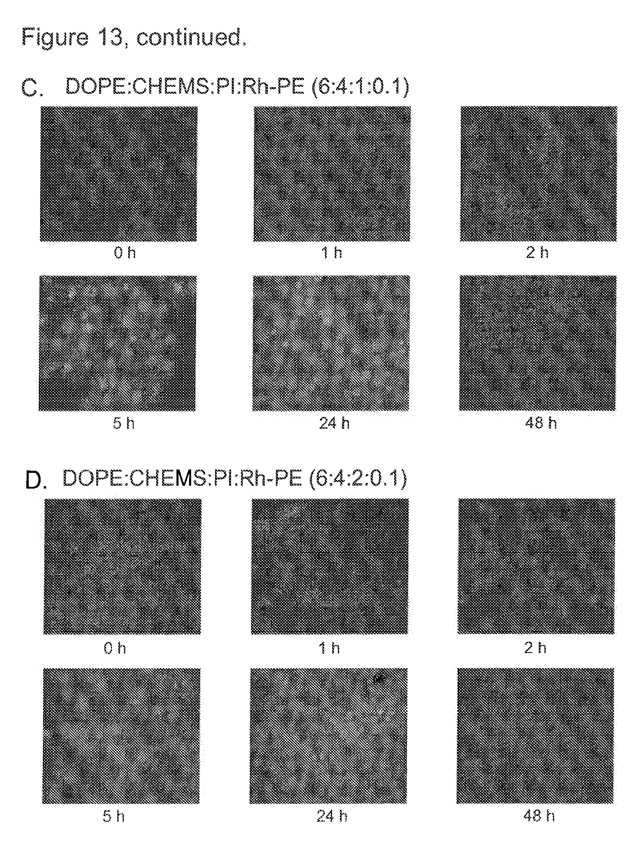

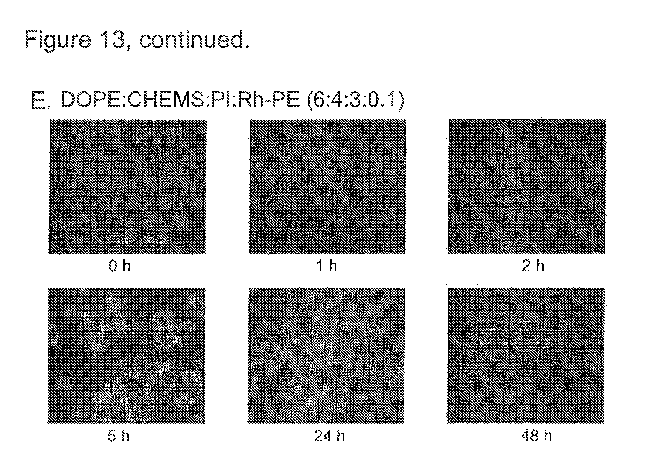

FIG. 13 A-E show representative fluorescent images of rhodamine labelled PE inside MDBK cells following a 15 m pulse with various liposome preparations. In particular, FIG. 13A demonstrates data for DOPE:CHEMS:Rh-PE (6:4:0.1); FIG. 13B for DOPC:CHEMS:Rh-PE (6:4:0.1); FIG. 13C for DOPE:CHEMS:PI:Rh-PE (6:4:1:0.1); FIG. 13D DOPE:CHEMS:PI:Rh-PE (6:4:2:0.1) and FIG. 13E DOPE:CHEMS:PI:Rh-PE (6:4:3:0.1). Intracellular localization of Rh-PE was observed for each liposome preparation at time points: 0, 1, 2, 5, 24 and 48 hours. DAPI counterstain was used to visualize all cells

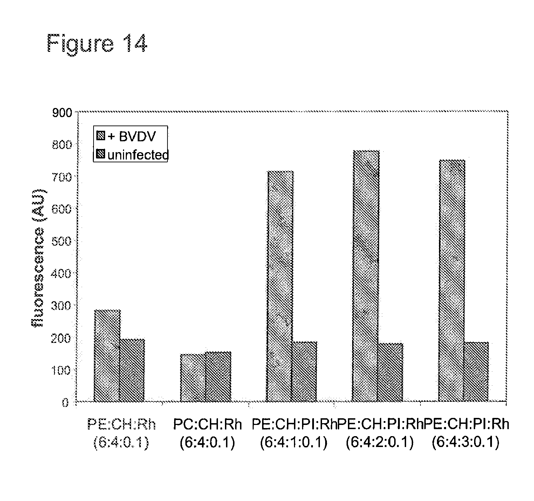

FIG. 14 shows results from treating both cp-BVDV-infected and uninfected MDBK cells with various liposome preparations containing Rh-labelled PE and measuring the secretion of the Rh-PE lipid following three days incubation. An increase in the Rh-PE secretion between infected and uninfected cells treated with the same liposome composition was due to secretion of viral particles, which have budded from the ER membrane, containing the Rh-PE lipid.

### DETAILED DESCRIPTION

Unless otherwise specified, "a" or "an" means "one or more." Definition of terms:

As used herein, the term "viral infection" describes a diseased state, in which a virus invades a healthy cell, uses the cell's reproductive machinery to multiply or replicate and ultimately lyse the cell resulting in cell death, release of viral particles and the infection of other cells by the newly produced progeny viruses. Latent infection by certain viruses is also a possible result of viral infection.

As used herein, the term "treating or preventing viral infection" means to inhibit the replication of the particular virus, to inhibit viral transmission, or to prevent the virus from establishing itself in its host, and to ameliorate or alleviate the symptoms of the disease caused by the viral infection. The treatment is considered therapeutic if there is a reduction in viral load, decrease in mortality and/or morbidity.

The term "therapeutic agent" refers to any agent, such as a molecule or a compound, which can assist in treating a physiological condition, such as a viral infection or a disease caused thereby.

The term "synergistic" as used herein refers to a combination, which is more effective than the additive effects of any two or more single therapeutic agents. A synergistic effect as used herein refers to the ability to use lower amounts (doses) of either single therapy to treat or prevent a physiological condition, such as a viral infection or a disease caused thereby. The lower doses can result in lower toxicity without reducing efficacy of the treatment. In addition, a synergistic effect can result in improved efficacy, e.g. in an improved antiviral activity. Finally, for a viral infection, synergy may result in an improved avoidance or reduction of a viral resistance against a single therapeutic agent or single therapy.

Liposomes can be defined as organic compounds comprising lipids in a spherical bilayer formation. Liposomes discussed herein may include one or more lipids represented by the following abbreviations:
CHEMS stands for cholesteryl hemisuccinate lipid.
DOPE stands for dioleoylphosphatidylethanolamine lipid.
DOPC stands for dioleoylphosphatidylcholine lipid.
PE stands for phosphatidylethanolamine lipid.
PEG-PE stands for polyethylene glycol (2000)-distearoylphosphatidylethanolamine lipid.
Rh-PE stands for lissamine rhodamine B-phosphatidylethanolamine lipid.
MCC-PE stands for 1,2-Dioleoyl-sn-Glycero-3-Phosphoethanolamine-N-[4-(p-maleimidomethyl)cyclohexane-carboxamide] lipid.
PI stands for phosphatidylinositol lipid.
The term "intracellular delivery" refers to the delivery of encapsulated material from liposomes into any intracellular compartment.
IC50 or IC90 (inhibitory concentration 50 or 90) is a concentration of a therapeutic agent used to achieve 50% or 90% reduction of viral infection, respectively.
A DC liposome designates a liposome comprising DOPE and CHEMS lipids with a molar ratio of 6:3.
A DCPP liposome designates a liposome comprising DOPE, CHEMS and PEG-PE lipids with a molar ratio of 6:4:0.3.
PBMC stands for peripheral blood mononuclear cell.
sCD4 stands for a soluble CD4 molecule. By "soluble CD4" or "***sCD4***" or D1D2" is meant a CD4 molecule, or a fragment thereof, that is in aqueous solution and that can mimic the activity of native membrane-anchored CD4 by altering the conformation of HIV Env, as is understood by those of ordinary skill in the art. One example of a soluble CD4 is the two-domain soluble CD4 (***sCD4*** or D1D2) described, e.g., in Salzwedel et al. J. Virol. 74:326 333, 2000.
MAb stands for a monoclonal antibody.
DNJ denotes deoxynojirimycin.
*N*B-DNJ denotes *N-*butyl deoxynojirimycin.
*N*N-DNJ denotes *N-*nonyl deoxynojirimycin.
BVDV stands for bovine viral diarrhea virus.
HBV stands for hepatitis B virus.
HCV stands for hepatitis C virus.
HIV stands for human immunodeficiency virus.
Ncp stands for non-cytopatic.
Cp stands for cytopatic.
ER stands for endoplasmic reticulum.
CHO stands for Chinese hamster ovary cells
MDBK stands for Madin-Darby bovine kidney cells.
PCR stands for polymerase chain reaction.
FOS stands for free oligosaccharides.
HPLC stands for high performance liquid chromatography.
PHA stands for phytohemagglutinin.
FBS stands for fetal bovine serum.
TCID50 stands for 50% tissue culture infective dose.
ELISA stands for Enzyme Linked Immunosorbent Assay.
IgG stands for immunoglobuline.
DAPI stands for 4',6-Diamidino-2-phenylindole.
PBS stands for phosphate buffered saline.

### LIPOSOMES TREATMENT OF VIRAL INFECTIONS

The inventors have discovered that, upon contacting a cell, a pH sensitive liposome, that comprises DOPE, CHEMS and/or PEG-PE lipids, such as a DC liposome or a DCPP liposome, can be able to bypass the cell's endosomal pathway following endocytosis and deliver a material encapsulated in the liposome directly into the cell's endoplasmic reticulum (ER), i.e. in the ER lumen. One or more lipids of the liposome can also integrate into the ER membrane of the cell. This discovery can have a major implication for the treatment of viral infections, for which the virus requires budding from the ER membrane, such as HBV, HCV and BVDV infections, as incorporation of liposome lipids with the ER membrane of a cell infected with the virus can alter the envelope of budding virus particles and, thus, reduce infectivity.

The inventors have also discovered that encapsulation of *N-*butyl deoxynojirimycin (*N*B-DNJ) in a DCPP liposome can provide an increased intracellular delivery as compared to DCPP encapsulation of other deoxynojirimycin compounds, such as deoxynojirimycin (DNJ) or *N-*nonyl deoxynojirimycin (*N*N-DNJ). Such an increased intracellular delivery of *N*B-DNJ can lead to an enhancement of *in vivo* activity of *N*B-DNJ.

Furthermore, the inventors have discovered that a liposome comprising DOPE, CHEMS and/or PEG-PE lipids, such as a DCPP liposome, can have an antiviral effect of its own, i.e. independent of any therapeutic agents encapsulated inside the liposome, and that the DCPP liposome and a therapeutic agent, such as *N*B-DNJ, that is encapsulated inside the liposome can act synergistically against the virus.

Accordingly, one embodiment is a method of treating an ER membrane virus budding infection, i.e. a viral infection, for which virus budding can occur at the ER membrane, such as HBV, HCV or BVDV infection. The method can involve contacting a cell infected with a virus responsible for the infection, with a composition, in which *N*B-DNJ is encapsulated in a liposome that comprises DOPE and CHEMS lipids. Such contacting can provide a synergistic therapy resulting in delivering one or more lipids of the liposome to the ER membrane of the contacted cell, altering the membrane and thereby reducing infectivity of progeny viruses, and by releasing the encapsulated *N*B-DNJ directly into the cell's ER lumen.

Another embodiment is a method of treating a viral infection by contacting a cell, that is infected with a virus responsible for the infection, with a composition that contains 1) a liposome comprising DOPE, CHEMS and PEG-PE lipids and 2) a therapeutic agent that is encapsulated inside the liposome. The viral infection can be, for example, HCV, HBV, BVDV, HIV, Moloney murine leukaemia virus, murine hepatitis virus, herpes simplex virus types 1 and 2, cytomegalovirus, Sindbis virus, Semliki forest virus, Vesicular stomatis virus, Influenza A virus, Measles virus, Dengue virus, or Japanese Encephalitis virus, as described in R. A. Dwek, et al, Nat. Rev. Drug Discov. 2002 Jan; 1(1):65-75.

In some embodiments, the therapeutic agent encapsulated inside the liposome can be, an α-glucosidase inhibitor. In some embodiments, the α-glucosidase inhibitor can be ER α-glucosidase inhibitor. In general, any virus that relies on interactions with calnexin and/or calreticulin for proper folding of its viral envelope glycoproteins, can be targeted with ER α-glucosidase inhibitor.

The alpha-glucosidase inhibitor can be an agent that inhibits host alpha-glucosidase enzymatic activity by at least about 10%, at least about 15%, at least about 20%, at least about 25%, at least about 30%, at least about 35%, at least about 40%, at least about 50%, at least about 60%, at least about 70%, at least about 80%, or at least about 90%, or more, compared to the enzymatic activity of the alpha-glucosidase in the absence of the agent. The term "alpha-glucosidase inhibitot" encompasses both naturally occuring and synthetic agents that inhibit host alpha-glucosidase activity.

Suitable alpha-glucosidase inhibitors include, but not limited to, deoxynojirimycin and N-substituted deoxynojirimycins, such as compounds of Formula II and pharmaceutically acceptable salts thereof:

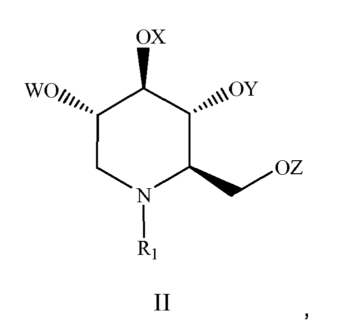

where R₁ is selected from substituted or unsubstituted alkyl groups, substituted or unsubstituted cycloalkyl groups, substituted or unsubstituted aryl groups, or substituted or unsubstituted oxaalkyl groups, selected from but not limited to arylalkyl, cycloalkylalkyl, branched or straight chain alkyl groups, and oxaalkyl groups; and where W, X, Y, and Z are each independently selected from hydrogen, alkanoyl groups, aroyl groups, and haloalkanoyl groups.

In some of such embodiments, R1 is selected from ethyl, propyl, isopropyl, butyl, isobutyl, tert-butyl, pentyl, neopentyl, isopentyl, hexyl, -(CH₂)₂O(CH₂)₅CH₃, -(CH₂)₂O(CH₂)₆CH₃, -(CH₂)₆OCH₂CH₃, and -(CH₂)₂OCH₂CH₂CH₃. In other such embodiments, R1 is butyl, and W, X, Y, and Z are all hydrogen.

In some embodiments, the compound of Formula II is selected from, but is not limited to N-(n-hexyl-)-1,5-dideoxy-1,5-imino-D-glucitol; N-(n-heptyl-)-1,5-dideoxy-1,5-imino-D-glucitol; N-(n-octyl-)-1,5-dideoxy-1,5-imino-D-glucitol; N-(n-octyl-)-1,5-dideoxy-1,5-imino-D-glucitol, tetrabutyrate; N-(n-nonyl-)-1,5-dideoxy-1,5-imino-D-glucitol, tetrabutyrate; N-(n-decyl-)-1,5-dideoxy-1,5-imino-D-glucitol, tetrabutyrate; N-(n-undecyl-)-1,5-dideoxy-1,5-imino-D-glucitol, tetrabutyrate; N-(n-nonyl-)-1,5-dideoxy-1,5-imino-D-glucitol; N-(n-decyl-)-1,5-dideoxy-1,5-imino-D-glucitol; N-(n-undecyl-)-1,5-dideoxy-1,5-imino-D-glucitol; N-(n-dodecyl-)-1,5-dideoxy-1,5-imino-D-glucitol; N-(2-ethylhexyl)-1,5-dideoxy-1,5-imino-D-glucitol; N-(4-ethylhexyl)-1,5-dideoxy-1,5-imino-D-glucitol; N-(5-methylhexyl)-1,5-dideoxy-1,5-imino-D-glucitol; N-(3-propylhexyl)-1,5-dideoxy-1,5-imino-D-glucitol; N-(1-pentylpentylhexyl)-1,5-dideoxy-1,5-imino-D-glucitol; N-(1-butylbutyl)-1,5-dideoxy-1,5-imino-D-glucitol; N-(7-methyloctyl-)-1,5-dideoxy-1,5-imino-D-glucitol; N-(8-methylnonyl)-1,5-dideoxy-1,5-imino-D-glucitol; N-(9-methyldecyl)-1,5-dideoxy-1,5-imino-D-glucitol; N-(10-methylundecyl)-1,5-dideoxy-1,5-imino-D-glucitol; N-(6-cyclohexylhexyl-)-1,5-dideoxy-1,5-imino-D-glucitol; N-(4-cyclohexylbutyl)-1,5-dideoxy-1,5-imino-D-glucitol; N-(2-cyclohexylethyl)-1,5-dideoxy-1,5-imino-D-glucitol; N-(1-cyclohexylmethyl)-1,5-dideoxy-1,5-imino-D-glucitol; N-(1-phenylmethyl)-1,5-dideoxy-1,5-imino-D-glucitol; N-(3-phenylpropyl)-1,5-dideoxy-1,5-imino-D-glucitol; N-(3-(4-methyl)-phenylpropyl)-1,5-dideoxy-1,5-imino-D-glucitol; N-(6-phenylhexyl)-1,5-dideoxy-1,5-imino-D-glucitol; N-(n-nonyl-)-1,5-dideoxy-1,5-imino-D-glucitol, tetrabutyrate; N-(n-decyl-)-1,5-dideoxy-1,5-imino-D-glucitol, tetrabutyrate; N-(n-undecyl-)-1,5-dideoxy-1,5-imino-D-glucitol, tetrabutyrate; N-(n-dodecyl-)-1,5-dideoxy-1,5-imino-D-glucitol, tetrabutyrate; N-(2-ethylhexyl)-1,5-dideoxy-1,5-imino-D-glucitol, tetrabutyrate; N-(4-ethylhexyl)-1,5-dideoxy-1,5-imino-D-glucitol, tetrabutyrate; N-(5-methylhexyl)-1,5-dideoxy-1,5-imino-D-glucitol, tetrabutyrate; N-(3-propylhexyl)-1,5-dideoxy-1,5-imino-D-glucitol, tetrabutyrate; N-(1 -pentylpentylhexyl)-1,5-dideoxy-1,5-imino-D-glucitol, tetrabutyrate; N-(1-butylbutyl)-1,5-dideoxy-1,5-imino-D-glucitol, tetrabutyrate; N-(7-methyloctyl-)-1,5-dideoxy-1,5-imino-D-glucitol, tetrabutyrate; N-(8-methylnonyl)-1,5-dideoxy-1,5-imino-D-glucitol, tetrabutyrate; N-(9-methyldecyl)-1,5-dideoxy-1,5-imino-D-glucitol, tetrabutyrate; N-(10-methylundecyl)-1,5-dideoxy-1,5-imino-D-glucitol, tetrabutyrate; N-(6-cyclohexylhexyl-)-1,5-dideoxy-1,5-imino-D-glucitol, tetrabutyrate; N-(4-cyclohexylbutyl)-1,5-dideoxy-1,5-imino-D-glucitol, tetrabutyrate; N-(2-cyclohexylethyl)-1,5-dideoxy-1,5-imino-D-glucitol, tetrabutyrate; N-(1-cyclohexylmethyl)-1,5-dideoxy-1,5-imino-D-glucitol, tetrabutyrate; N-(1-phenylmethyl)-1,5-dideoxy-1,5-imino-D-glucitol, tetrabutyrate; N-(3-phenylpropyl)-1,5-dideoxy-1,5-imino-D-glucitol, tetrabutyrate; N-(3-(4-methyl)-phenylpropyl)-1,5-dideoxy-1,5-imino-D-glucitol, tetrabutyrate; N-(6-phenylhexyl)-1,5-dideoxy-1,5-imino-D-glucitol, tetrabutyrate; pharmaceutically acceptable salts thereof; and mixtures of any two or more thereof.

Suitable alpha-glucosidase inhibitors also include N-oxaalkylated deoxynojirimycins such as N-hydroxyethyl DNJ (Miglitol or Glyset® ) described in US patent 4,639,436.

Suitable alpha-glucosidase inhibitors also include castanospermines and castanospermine derivatives, such as compounds of Formula (I) and pharmaceutically acceptable salts thereof disclosed in US patent application No. 2006/0194835, including 6-O-butanoyl castanospermine (celgosivir), and compounds and pharmaceutically acceptable salt thereof of Formula II disclosed in PCT publication No. W001054692.

In some embodiments, the alpha glucosidase inhibitor can be acarbose (O-4,6-dideoxy-4-[[(1S,4R,5S, 6S)-4,5 ,6-trihydroxy-3-(hydroxymethyl)-2-cyc- lohexen-1-yl]amino]-α-D-glucopyranosyl-(1→4)-O-→-D-gluc- opyranosyl-(1→4)-D-glucose), or Precose®. Acarbose is disclosed in U.S. Pat. No. 4,904,769. In some embodiments, the alpha glucosidase inhibitor can be a highly purified form of acarbose (see, e.g., U.S. Pat. No. 4,904,769).

In some embodiments, the therapeutic agent encapsulated inside the liposome can be an ion channel inhibitor. In some embodiments, the ion channel inhibitor can be an agent inhibiting the activity of HCV p7 protein. Ion channel inhibitors and methods of identifying them are detailed in US patent publication 2004/0110795. Suitable ion channel inhibitors include compounds of Formula I and pharmaceutically acceptable salts thereof, including N-(7-oxa-nonyl)-1,5,6-trideoxy-1,5-imino-D-galactitol (N-7-oxa-nonyl 6-MeDGJ or UT231B) and N-10-oxaundecul-6-MeDGJ. Suitable ion channel inhibitors also include, but not limited to, N-nonyl deoxynojirimycin, N-nonyl deoxynogalactonojirimycin and N-oxanonyl deoxynogalactonojirimycin.

In some embodiments, the therapeutic agent encapsulated inside the liposome can include an iminosugar. Suitable iminosugars include both naturally occurring iminosugars and synthetic iminosugars.

In some embodiments, the iminosugar can be deoxynojirimycin or N-substituted deoxynojirimycin derivative. Examples of suitable N-substituted deoxynojirimycin derivatives include, but not limited to, compounds of Formula II of the present application, compounds of Formula I of US patent No. 6,545,021 and N-oxaalkylated deoxynojirimycins, such as N-hydroxyethyl DNJ (Miglitol or Glyset® ) described in US patent 4,639,436.

In some embodiments, the iminosugar can be castanospermine or castanospermine derivative. Suitable castanospemine derivatives include, but not limited to, compounds of Formula (I) and pharmaceutically acceptable salts thereof disclosed in US patent application No. 2006/0194835 and compounds and pharmaceutically acceptable salt thereof of Formula II disclosed in PCT publication No. WO0105469

In some embodiments, the iminosugar can be deoxynogalactojirimycin or N-substituted derivative thereof such as those disclosed in PCT publications No. WO99/24401 and WO01/10429. Examples of suitable N-substituted deoxynogalactojirimycin derivatives include, but not limited to, N-alkylated deoxynogalactojirimycins (N-alkyl-1,5-dideoxy-1,5-imino-D-galactitols), such as N-nonyl deoxynogalactojirimycin, and N-oxa-alkylated deoxynogalactojirimycins (N-oxa-alkyl-1,5-dideoxy-1,5-imino-D-galactitols), such as N-7-oxanonyl deoxynogalactojirimycin.

In some embodiments, the iminosugar can be N-substituted 1,5,6-trideoxy-1,5-imino-D-galactitol (N-substituted MeDGJ) including, but not limited to compounds of Formula I:

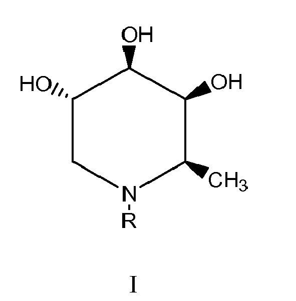

wherein R is selected from substituted or unsubstituted alkyl groups, substituted or unsubstituted cycloalkyl groups, substituted or unsubstituted heterocyclyl groups, or substituted or unsubstituted oxaalkyl groups. In some embodiments, substituted or unsubstituted alkyl groups and/or substituted or unsubstituted oxaalkyl groups comprise from 1 to 16 carbon atoms, or from 4 to 12 carbon atoms or from 8 to 10 carbon atoms. In some embodiments, substituted or unsubstituted alkyl groups and/or substituted or unsubstituted oxaalkyl groups comprise from 1 to 4 oxygen atoms, and from 1 to 2 oxygen atoms in other embodiments. In other embodiments, substituted or unsubstituted alkyl groups and/or substituted or unsubstituted oxaalkyl groups comprise from 1 to 16 carbon atoms and from 1 to 4 oxygen atoms. Thus, in some embodiments, R is selected from, but is not limited to -(CH₂)₆OCH₃, -(CH₂)₆OCH₂CH₃, -(CH₂)₆O(CH₂)₂CH₃, -(CH₂)₆O(CH₂)₃CH₃, -(CH₂)₂O(CH₂)₅CH₃, -(CH₂)₂O(CH₂)₆CH₃, and -(CH₂)₂O(CH₂)₇CH₃. N-substituted MeDGJs are disclosed, for example, in PCT publication No. WO01/10429. In some embodiments, the therapeutic agent encapsulated inside the liposome can include a nitrogen containing compound having formula VIII or a pharmaceutically acceptable salt thereof: 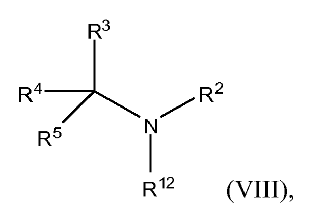 wherein R¹² is an alkyl such as C₁-C₂₀, or C₁-C₆ or C₇-C₁₂ or C₈-C₁₆ and can also contain from 1 to 5 or from 1 to 3 or from 1 to 2 oxygen, R¹² can be an oxa-substituted alkyl derivative. Examples if oxa-substituted alkyl derivatives include 3-oxanonyl, 3-oxadecyl, 7-oxanonyl and 7-oxadecyl.
R² is hydrogen, R³ is carboxy, or a C₁-C₄ alkoxycarbonyl, or R² and R³, together are 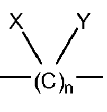 or -(CXY)ₙ-, wherein n is 3 or 4, each X, independently, is hydrogen, hydroxy, amino, carboxy, a C₁-C₄ alkylcarboxy, a C₁-C₄ alkyl, a C₁-C₄ alkoxy, a C₁-C₄ hydroxyalkyl, a C₁-C₆ acyloxy, or an aroyloxy, and each Y, independently, is hydrogen, hydroxy, amino, carboxy, a C₁-C₄ alkylcarboxy, a C₁-C₄ alkyl, a C₁-C₄ alkoxy, a C₁-C₄ hydroxyalkyl, a C₁-C₆ acyloxy, an aroyloxy, or deleted (i.e. not present);
R⁴ is hydrogen or deleted (i.e. not present); and R⁵ is hydrogen, hydroxy, amino, a substituted amino, carboxy, an alkoxycarbonyl, an aminocarbonyl, an alkyl, an aryl, an aralkyl, an alkoxy, a hydroxyalkyl, an acyloxy, or an aroyloxy, or R³ and R⁵, together, form a phenyl and R⁴ is deleted (i.e. not present).
In some embodiments, the nitrogen containing compound has the formula: 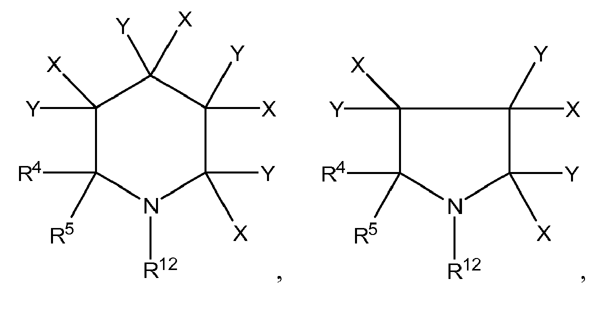 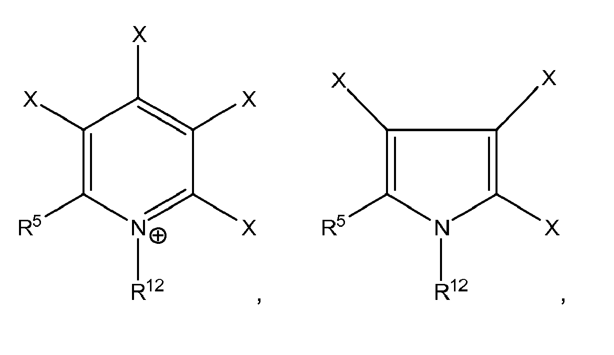 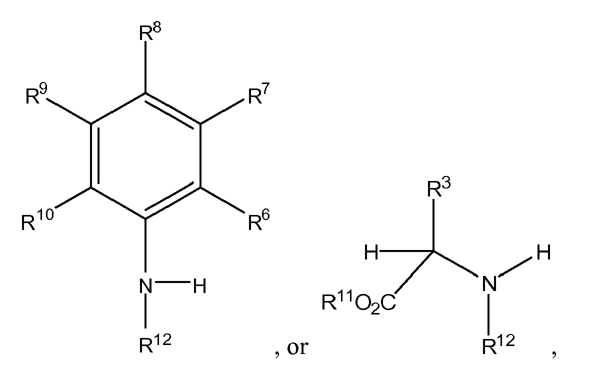 where each of R⁶-R¹⁰, independently, is selected from the group consisting of hydrogen, hydroxy, amino, carboxy, C₁-C₄ alkylcarboxy, C₁-C₄ alkyl, C₁-C₄ alkoxy, C₁-C₄ hydroxyalkyl, C₁-C₄ acyloxy, and aroyloxy; and R¹¹ is hydrogen or C₁-C₆ alkyl. The nitrogen-containing compound can be N-alkylated piperidine, N-oxa-alkylated piperidine, N-alkylated pyrrolidine, N-oxa-alkylated pyrrolidine, N-alkylated phenylamine, N-oxa-alkylated phenylamine, N-alkylated pyridine, N-oxa-alkylated pyridine, N-alkylated pyrrole, N-oxa-alkylated pyrrole, N-alkylated amino acid, or N-oxa-alkylated amino acid. In certain embodiments, the N-alkylated piperidine, N-oxa-alkylated piperidine, N-alkylated pyrrolidine, or N-oxa-alkylated pyrrolidine compound can be an iminosugar. For example, in some embodiments, the nitrogen-containing compound can be N-alkyl-1,5-dideoxy-1,5-imino-D-galactitol (N-alkyl-DGJ) or N-oxa-alkyl-1,5-dideoxy-1,5-imino-D-galactitol (N-oxa-alkyl-DGJ) having the formula: 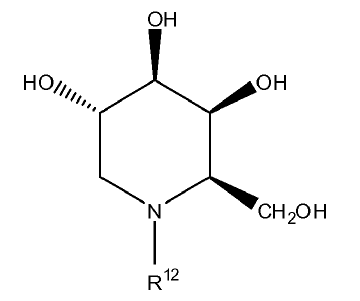 or N-alkyl-1,5,6-trideoxy-1,5-imino-D-galactitol (N-alkyl-MeDGJ) or N-oxa-alkyl-1,5,6-trideoxy-1,5-imino-D-galactitol having (N-oxa-alkyl-MeDGJ) having the formula:

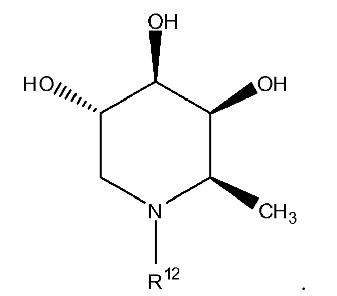

As used herein, the groups have the following characteristics, unless the number of carbon atoms is specified otherwise. Alkyl groups have from 1 to 20 carbon atoms and are linear or branched, substituted or unsubstituted. Alkoxy groups have from 1 to 16 carbon atoms, and are linear or branched, substituted or unsubstituted. Alkoxycarbonyl groups are ester groups having from 2 to 16 carbon atoms. Alkenyloxy groups have from 2 to 16 carbon atoms, from 1 to 6 double bonds, and are linear or branched, substituted or unsubstituted. Alkynyloxy groups have from 2 to 16 carbon atoms, from 1 to 3 triple bonds, and are linear or branched, substituted or unsubstituted. Aryl groups have from 6 to 14 carbon atoms (e.g., phenyl groups) and are substituted or unsubstituted. Aralkyloxy (e.g., benzyloxy) and aroyloxy (e.g., benzoyloxy) groups have from 7 to 15 carbon atoms and are substituted or unsubstituted. Amino groups can be primary, secondary, tertiary, or quaternary amino groups (i.e., substituted amino groups). Aminocarbonyl groups are amido groups (e.g., substituted amido groups) having from 1 to 32 carbon atoms. Substituted groups can include a substituent selected from the group consisting of halogen, hydroxy, C₁-₁₀ alkyl, C₂-₁₀ alkenyl, C₁-₁₀ acyl, or C₁-₁₀ alkoxy.

The N-alkylated amino acid can be an N-alkylated naturally occurring amino acid, such as an N-alkylated a-amino acid. A naturally occurring amino acid is one of the 20 common a-amino acids (Gly, Ala, Val, Leu, Ile, Ser, Thr, Asp, Asn, Lys, Glu, Gln, Arg, His, Phe, Cys, Trp, Tyr, Met, and Pro), and other amino acids that are natural products, such as norleucine, ethylglycine, ornithine, methylbutenyl-methylthreonine, and phenylglycine. Examples of amino acid side chains (e.g., R⁵) include H (glycine), methyl (alanine), -CH₂C(O)NH₂ (asparagine), -CH₂-SH (cysteine), and -CH(OH)CH₃ (threonine).

An N-alkylated compound can be prepared by reductive alkylation of an amino (or imino) compound. For example, the amino or imino compound can be exposed to an aldehyde, along with a reducing agent (e.g., sodium cyanoborohydride) to N-alkylate the amine. Similarly, a N-oxa-alkylated compound can be prepared by reductive alkylation of an amino (or imino) compound. For example, the amino or imino compound can be exposed to an oxa-aldehyde, along with a reducing agent (e.g., sodium cyanoborohydride) to N-oxa-alkylate the amine.

The nitrogen-containing compound can include one or more protecting groups. Various protecting groups are well known. In general, the species of protecting group is not critical, provided that it is stable to the conditions of any subsequent reaction(s) on other positions of the compound and can be removed at the appropriate point without adversely affecting the remainder of the molecule. In addition, a protecting group may be substituted for another after substantive synthetic transformations are complete. Clearly, where a compound differs from a compound disclosed herein only in that one or more protecting groups of the disclosed compound has been substituted with a different protecting group, that compound is within the invention. Further examples and conditions are found in Greene, Protective Groups in Organic Chemistry, (1st Ed., 1981, Greene & Wuts, 2nd Ed., 1991).

The nitrogen-containing compound can be purified, for example, by crystallization or chromatographic methods. The compound can be prepared stereospecifically using a stereospecific amino or imino compound as a starting material.

The amino and imino compounds used as starting materials in the preparation of the long chain N-alkylated compounds are commercially available (Sigma, St. Louis, MO; Cambridge Research Biochemicals, Norwich, Cheshire, United Kingdom; Toronto Research Chemicals, Ontario, Canada) or can be prepared by known synthetic methods. For example, the compounds can be N-alkylated imino sugar compounds or oxa-substituted derivatives thereof. The imino sugar can be, for example, deoxygalactonojirmycin (DGJ), 1-methyl-deoxygalactonojirimycin (MeDGJ), deoxynorjirimycin (DNJ), altrostatin, 2R,5R-dihydroxymethyl-3R,4R-dihydroxypyrrolidine (DMDP), or derivatives, enantiomers, or stereoisomers thereof.

The syntheses of a variety of iminosugar compounds have been described. For example, methods of synthesizing DNJ derivatives are known and are described, for example, in U.S. Patent Nos. 5,622,972, 5,401,645, 5,200,523, 5,043,273, 4,994,572, 4,246,345, 4,266,025, 4,405,714, and 4,806,650. Methods of synthesizing other iminosugar derivatives are known and are described, for example, in U.S. Patent Nos. 4,861,892, 4,894,388, 4,910,310, 4,996,329, 5,011,929, 5,013,842, 5,017,704, 5,580,884, 5,286,877, and 5,100,797 and PCT publication No. WO 01/10429. The enantiospecific synthesis of 2*R,*5*R*-dihydroxymethyl-3*R*,4*R*-dihydroxypyrrolidine (DMDP) is described by Fleet & Smith (Tetrahedron Lett. 26:1469-1472, 1985).

The contacted cell can be a cell from a mammal, such as a human. In some cases, contacting the infected cell with the liposome composition can be done through administering the composition to a subject that comprises the infected cell. The subject can be a mammal, such as a human. In some embodiments, the liposomal composition can be administered by intravenous injection. Yet in some embodiments, the liposomal composition can be administered via a parenteral routes other than intravenous injection, such as intraperitoneal, subcutaneous, intradermal, intraepidermal, intramuscular or transdermal route. Yet in some embodiments, the liposomal composition can be administered via a mucosal surface, e.g. an ocular, intranasal, pulmonary, intestinal, rectal and urinary tract surfaces. Administration routes for liposomal compositions are disclosed, for example, in A. S. Ulrich, Biophysical Aspects of Using Liposomes as Delivery Vehicles, Bioscience Reports, Volume 22, Issue 2, Apr 2002, 129 - 150.

Delivery of a therapeutic agent, such as *N*B-DNJ, via the liposome into the ER lumen can lower an effective amount of the therapeutic agent required for inhibition of ER-glucosidase compared to non-liposome methods. For example, for *N*B-DNJ, the IC90 can be reduced by at least 100, or by at least 500, or by at least 1000, or by at least 5000, or by at least 10000, or by at least 50000 or by at least 100000. Such a reduction of the effective antiviral amount of NB-DNJ can result in final concentrations of administered NB-DNJ that are one or more orders of magnitude below toxic levels in mammals, in particular, humans.

In some cases, the liposome composition comprising a therapeutic agent, such as *N*B-DNJ, can be contacted with the infected cell in combination with one or more additional therapeutic agents, such as antiviral agents. In some cases, such additional therapeutic agents can be co-encapsulated with *N*B-DNJ into the liposome. Yet in some cases, contacting the infected cell with such additional therapeutic agents can be a result of administering the additional therapeutic agents to a subject comprising the cell. The administration of the additional therapeutic agents can be carried out by adding the therapeutic agents to the composition. Yet in some cases, the administration of the additional therapeutic agents can be performed separately from administering the liposome composition containing *N*B-DNJ. Such separate administration can be performed via an administration pathway that can the same or different that the administration pathway used for the liposome composition.

Combination therapy may not only reduce the effective dose of an agent required for antiviral activity, thereby reducing its toxicity, but may also improve the absolute antiviral effect as a result of attacking the virus through multiple mechanisms. For example, lipids of the DCPP liposome and *N*B-DNJ can act 1) at an envelope of the virus, where treatment with *N*B-DNJ containing liposomes can alter the envelope's composition with the addition of foreign lipids and 2) through misfolding of viral glycoproteins, thereby reducing the infectivity. Thus, the liposome encapsulating *N*B-DNJ used in combination with one or more agents, that has targets or mechanisms of action different from *N*B-DNJ, can provide additive or synergistic effect.

In addition, combination therapy can provide means for circumventing or decreasing a chance of development of viral resistance.

The particular additional therapeutic agent(s) that can be used in combination the liposome containing *N*B-DNJ can depend of the viral infection being treated. For example, for a hepatitis infection, such as HBV, HCV or BVDV infection, such therapeutic agent(s) can be a nucleoside or nucleotide antiviral agent and/or an immunostimulating/ immunomodulating agent. Various nucleoside agents, nucleotide agents and immunostimulating/ immunomodulating agents that can be used in combination with *N*B-DNJ for treatment of hepatitis are exemplified in US patent No. 6,689,759 issued February 10, 2004, to Jacob *et. al.* For example, for treatment of hepatitis C infection, *N*B-DNJ can be encapsulated in the liposome in combination with 1-b-D-ribofuranosyl-1H-1,2,4-triazole-3-carboxamide (ribavirin), as a nucleoside agent, and interferon such as interferon alpha, as an immunostimulating/ immunomodulating agent. The treatment of hepatitis infections with ribavirin and/or interferon is discussed, for example, in US patents Nos. 6,172,046; 6,177,074; 6,299,872; 6,387,365; 6,472,373; 6,524,570 and 6,824,768.

For treating an HIV infection, a therapeutic agent that can be used in combination with a liposome containing *N*B-DNJ can be an anti-HIV agent, which can be, for example, nucleoside Reverse Transcriptase (RT) inhibitor, such as (-)-2'-deoxy-3'-thiocytidine-5'-triphosphate (3TC); (-)-cis-5-fluoro-1-[2-(hydroxy-methyl)-[1,3-oxathiolan-5-yl]cytosine (FTC); 3'-azido-3'-deoxythymidine (AZT) and dideoxy-inosine (ddI); a non-nucleoside RT inhibitors, such as N11-cyclopropyl-4-methyl-5,11-dihydro-6H-dipyrido[3,2-b:2'3'-e]-[1,4] diazepin-6-one (Neviparine), a protease inhibitor or a combination thereof. Anti HIV therapeutic agents can be used in double or triple combinations, such as AZT, DDI, and nevirapin combination.

### LIPOSOMES CONJUGATED WITH gp120/gp41 TARGETTING MOIETY

The invention also provides a composition comprising a liposome conjugated with a gp120/gp41 targeting moiety and a method of treating an HIV infection by contacting an cell infected with the infection with such composition. The gp120/gp41 targeting moiety can comprise a sCD4 molecule or a monoclonal antibody, such as IgG 2F5 or IgG b12 antibodies. In some embodiments, the liposome can comprise DOPE and CHEMS lipids. In some embodiments, the liposome can further comprise PEG-PE lipids. In some embodiments, the liposome can further comprise MCC-PE lipids. For treatment of the HIV infection, the composition can further comprise an additional therapeutic agent, such as *N*B-DNJ, encapsulated inside the liposome.

### LIPOSOMES COMPRISING PI LIPIDS

The inventors have also discovered that a liposome, such as a pH sensitive liposome, that comprises phosphatidylinositol (PI) lipids, can target more effectively the ER membrane of a cell that a liposome that does not contain PI lipids. Furthermore, the liposome comprising PI lipids can increase a lifetime in the cell of one or more lipids delivered via the liposome. Accordingly, in some embodiments, the invention provides a composition that includes a liposome comprising PI lipids and at least one therapeutic agent, such as an antiviral therapeutic agent, encapsulated inside the liposome and a method for targeted delivery comprising administering such a composition to a subject, which can be a mammal, such as a human. Such a targeted delivery method can be used for treating or preventing a physiological condition, such as a viral infection or a disease caused thereof, in a subject affected by the condition.

Inventors further believe that incorporation of the at least one antiviral protein into ER-targeting liposome may synergistically reduce viral infectivity due to 1) direct delivery of lipids of the liposome into the ER membrane that when incorporated into viral envelope can reduce viral infectivity and 2) direct delivery of the at least one antiviral protein into the ER membrane that can incorporate into the viral envelope of budding particles and independently reduce infectivity. The encapsulation of at least one therapeutic agent, such as a antiviral therapeutic agent, into the liposome can provide additional synergistic effect due to 3) direct delivery of the therapeutic agent into intracellular compartments, such as ER lumen.

Accordingly, in some embodiments, the invention provides a composition that includes a liposome comprising PI lipids and at least one protein, such as an antiviral protein, intercalated into a lipid bilayer of the liposome and a method for targeted delivery comprising administering such a composition to a subject, which can be a mammal, such as a human. Such a targeted delivery method can be used for treating or preventing a physiological condition, such as a viral infection or a disease caused thereof, in a subject affected by the condition.

Yet in some embodiments, the invention provides a composition that includes a liposome comprising PI lipids, at least one therapeutic agent, such as an antiviral composition, encapsulated inside the liposome and at least one protein, such as an antiviral protein, intercalated into a lipid bilayer of the liposome and a method for targeted delivery comprising administering such a composition to a subject, which can be a mammal, such as a human.

The viral infection can be, for example, an ER membrane budding viral infection, i.e. a viral infection, for which virus budding occurs at the ER membrane, such as HBV, HCV or BVDV infection, or a plasma membrane budding viral infection, i.e. a viral infection, for which virus budding occurs at the plasma membrane, such as an HIV infection.

The liposome comprising PI lipids can further contain one or more lipids such as DOPE, CHEMS and/or PEG-PE lipids. A molar concentration of PI lipids in the liposome can vary from about 3% to about 60% or from about 5% to about 50% or from about 10% to about 30%.

In some embodiments, the therapeutic agent encapsulated inside the liposome comprising the PI lipids can be α-glucosidase inhibitor, such as any of α-glucosidase inhibitors discussed above.

In some embodiments, the therapeutic agent encapsulated inside the liposome comprising the PI lipids can be an ion channel activity inhibitor discussed above.

In some embodiments, the therapeutic agent encapsulated inside the liposome comprising the PI lipids can be an iminosugar, such as any of the iminosugars discussed above.

In some embodiments, the therapeutic agent encapsulated inside the liposome comprising the PI lipids can be a nitrogen containing compound of formula VIII.

In some embodiments, a protein intercalated into a liposome containing PI lipids can be an antiviral protein. Suitable antiviral proteins include, but are not limited to, viral receptors known to bind viral envelope proteins and/or mutated forms of viral envelope proteins and/or proteins known to interfere with viral envelope interactions. For example, for an HIV infection, the antiviral protein can be a CD4 protein. For HCV, BVDV, or HBV infections, the antiviral protein can be a mutated version of one of their respective viral envelope proteins, such as E1 or E2 proteins for HCV/BVDV.

The invention is further illustrated by, though in no way limited to, the following examples.

### LIPOSOME PREPARATION

Liposomes used in all experiments were prepared as follows: chloroform solutions of lipids were placed into glass tubes, and the solvent was evaporated under a stream of nitrogen followed by vacuum centrifugation under reduced pressure. Lipid films (10 µmoles total lipid) were hydrated by vortexing in 1 ml PBS buffer, pH 7.4 (with or without drug and/or with 80 mM calcein in PBS) for 1 h at room temperature. The resulting multilamellar vesicles were sonicated in a bath-type sonicator for 15 min followed by extrusion 21 times through a polycarbonate filter of 80-nm pore diameter.

### OPTIMIZATION OF ENCAPSULANT CONCENTRATION

The fluorescent molecule, calcein, was encapsulated inside DCPP liposomes at a concentration of 80 mM, at which concentration its fluorescence is self-quenched. Leakage of calcein from the liposomes, and its dilution into the surrounding medium, results in dequenching and an increase in measurable/observable fluorescence. Percent encapsulation was determined by diluting final liposome preparations containing calcein in MES-buffered saline, pH 7.4, to a final phospholipid concentration of 0.5 µM, and calcein was measured at λₑₓ = 490 and λₑₘ = 520 nm, before and after the addition of Triton X-100 to a final concentration of 0.1%. The difference in fluorescence following the addition of detergent is taken as the percent encapsulation within liposomes, and is used to estimate the amount of DNJ-compounds to determine the final concentrations of compounds in each experiment.

### LIPID ENCAPSULATION INTO THE ER MEMBRANE

Liposomes are able to deliver encapsulated material directly into the lumen of the ER while lipids are incorporated into the ER membrane. Initial evidence comes from tagging liposomes with a fluorescent label, where rhodamine conjugated to PE (Rh-PE) was incorporated into DCPP liposomes (1 % of molar content), so that lipid content was DOPE:CHEMS:PEG-PE:Rh-PE (DCPP-Rh), molar ratio of 6:4:0.3:0.1. MDBK cells were pulsed for 30 min with Rh-PE-labeled and calcein containing pH-sensitive liposomes. Unadsorbed liposomes were removed and the cells were further chased for 3 h. At the end of the chase period, cells were incubated for 30 min with ER-Tracker, an ER marker, washed and visualized. The merged picture shown in Figure 1 demonstrates that calcein, the Rh-labelled lipid and the ER tracker co-localize. Based on this, one can conclude that liposomes deliver their aqueous content to the ER, after an initial fusion step with the ER membrane shown by the co-localization of the liposomes lipids with the ER-tracker, a dye which integrates within the ER membrane.

### LIPOSOME TOXICITY

The toxicity of liposomes are measured in two different cell lines: Chinese hamster ovary (CHO) and Madin-Darby bovine kidney (MDBK) cells.

Cells were seeded in 6-well plates to over 80 % confluency, and DCPP liposomes encapsulating PBS were added to the media with a final lipid concentration ranging 0 - 500 µM for CHO cells, and 0 - 150 µM for MDBK cells. Cells and liposomes were left to incubate at 37 °C for 5 days before cells were harvested and counted following staining with trypan blue. Results are expressed as the percentage of viable cells present in treated samples as compared to the untreated control (= 100% on the x axis), and are the mean ± S.D. of duplicates from three separate experiments.

Results are demonstrated in Figure 2, where cytotoxicity in CHO cells gradually appeared following incubation in the presence of 150-µM lipid concentrations. MDBK cells appeared to be more sensitive to the DCPP liposomes, and demonstrated severe cytotoxicity at lipid concentrations greater than 75 µM.

### IMINOSUGAR RELEASE FROM DCPP LIPOSOMES

In all experiments with CHO and MDBK cells, DCPP liposomes have been added to a final lipid concentration of 100 µM and 50 µM, respectively (> 95% cell viability for both).

In the following example, the butyl chain of *N*B-DNJ is shown to be directly responsible for the increased intracellular release of encapsulated material from DCPP liposomes.

The effects of different DNJ compounds on the cellular uptake and intracellular release, when encapsulated inside DCPP liposomes, was determined by diluting compounds in 80-mM calcein and incorporating Rh-PE in liposome membranes as previously described, with the final liposome composition being DOPE:CHEMS:PEG-PE:Rh-PE (DCPP-Rh, 6:4:0.3:0.1) Liposomes encapsulating calcein alone, 10µ-M DNJ, 1-mM DNJ, 10-µM *N*B-DNJ, 1-mM *N*B-DNJ, 10-µM *N*N-DNJ, or 1-mM *N*N-DNJ, were incubated with CHO cells for 45 min before liposomes were removed and cells were washed twice in 1 x PBS. Cells were then analyzed in a fluorometer to measure calcein dequenching (λₑₓ = 490 and λₑₘ = 520 nm) and rhodamine fluorescence (λₑₓ = 584 and λₑₘ = 612 nm). Data represent the mean ± S.D. obtained from triplicates of four independent experiments, where mean rhodamine fluorescence values reflect the binding and uptake of liposomes and the mean calcein fluorescence reflects the intracellular dequenching of the dye (i.e. dye released from liposomes into intracellular compartments). Results are expressed as the percent fluorescence measured following incubation with DNJ-containing liposomes as compared to the calcein/PBS-only liposome control (= 100% on the x axis).

As shown in Figure 3, DNJ and *N*N-DNJ both had no effect on the amount of calcein dequenching, and, therefore, on intracellular release. *N*B-DNJ, however, demonstrates a concentration-dependent increase in dequenched calcein, where 10-µM and 1-mM formulations result in approximate 1.8- and 2.3-fold increases, respectively.

The calculated ratio of calcein to rhodamine fluorescence is taken as a measure of the amount of aqueous marker released per cell-associated liposome, and represents the efficiency of intracellular delivery. Efficiencies calculated for each encapsulated material are listed in Table 1. Results demonstrate that 10 µM and 1 mM *N*B-DNJ-containing liposomes increased the efficiency of intracellular delivery from DCPP liposomes by approximately 2- and 3-fold, respectively.

**Table 1. Efficiency of intracellular release from DCPP liposomes determined by the fluorescent measurements of dequenched calcein and Rh-PE incorporation taken from Figure 4.**

| Encapsulated Material | Efficiency (calcein/rhodamine, AU) |
|---|---|
| calcein only | 10.5±2.1 |
| 10 µM DNJ | 10.4±1.8 |
| 1 mM DNJ | 10.8±2.0 |
| 10 µM *N*B-DNJ | 21.7±1.5 |
| 1 mM *N*B-DNJ | 28.4±2.4 |
| 10 µM *N*N-DNJ | 4.4±1.3 |
| 1 mM *N*N-DNJ | 3.2±1.1 |

Although the present invention is not bound by its theory of operation, it is probable that a mechanism, by which the butyl chain of *N*B-DNJ promotes increased intracellular delivery of encapsulated material from DCPP liposomes, is through the destabilization of liposomes by insertion of the butyl chain within the lipid bilayer. The nine-carbon alkyl chain of *N*N-DNJ can also insert itself into the liposomes' lipid bilayer; however, at this length the molecule may actually stabilize the bilayer formation, as suggested by the presented results. Although *N*N-DNJ liposomes lead to an increase in cellular uptake, the amount of calcein released remains comparable to controls indicating increased liposome stability within the cell.

### STABILITY OF DCPP LIPOSOMES

In the following example the alkyl chains of both *N*B-DNJ and *N*N-DNJ are shown to change the properties of DCPP liposomes by making them more stable at lower pH.

DCPP liposomes encapsulating 1-mM DNJ, *N*B-DNJ, or *N*N-DNJ in 80-mM calcein buffer were compared with empty (calcein only) liposomes to determine the effects of the alkyl chain of *N*B-DNJ and *N*N-DNJ on the pH-sensitivity. 10 µl of calcein-loaded DCPP liposomes (final phospholipid concentration, 5 µM) was added to 2 ml of MES-buffered saline at various pH values ranging from 5.0 - 7.4, and left to incubate with shaking 15 min at 37 °C. Following incubation, calcein fluorescence was measured before and after the addition of Triton to a final concentration of 0.1%. Fluorescence intensities obtained at acidic pH values were corrected for the slight effect of pH on calcein fluorescence. The percentage of calcein release at each pH was calculated using the formula: % leakage = ((Iₙ-I₀)/(I₁₀₀-I₀)) x 100, where I₀ is the fluorescence at neutral pH, Iₙ is the corrected intensity at acidic pH before the addition of Triton, and I₁₀₀ is the total dequenched calcein at neutral pH.

Results are demonstrated in Figure 4, where no difference in stability between calcein only and DNJ encapsulated liposomes was observed. A significant decrease in pH sensitivity was observed, however, with *N*B-DNJ and *N*N-DNJ liposomes, indicating increased stability at low pH as a result of the alkyl chains. Although both *N*B-DNJ and *N*N-DNJ conferred greater stability to DCPP liposomes *in vitro,* only *N*N-DNJ actually inhibited calcein delivery *in vivo* (as observed in Figure 4 and Table 1), highlighting the presence of factors other than pH sensitivity that influence or control the process of intracellular cargo release.

Based on the findings from Figure 3, Figure 4 and Table 1, one can conclude that not only the presence, but also the length of the alkyl chain on the DNJ molecule can affect the properties of liposomes when encapsulated inside. These observations can be most likely a result of the alkyl chains inserting into the lipid bilayer of the liposomes, and effectively changing the lipid composition. Although only a 4 carbon chain (*N*B-DNJ) and a 9 carbon chain (*N*N-DNJ) have been used in these studies, it seems as though shorter chains can destabilize the liposomes *in vivo* leading to increased intracellular release of cargo molecules, whereas longer chains can stabilize the same lipid composition so that release is inhibited.

### DECREASE OF BVDV SECRETION BY NB-DNJ ENCAPSULATED IN DCPP LIPOSOMES

In the following example, *N*B-DNJ encapsulated in DCPP liposomes is shown to decrease the secretion of BVDV particles from MDBK cells.

The combined antiviral effects of *N*B-DNJ incorporated into DC and DCPP liposomes have been shown in MDBK cells infected with either the cp or ncp strain of BVDV.

In a first study using the ncp strain of BVDV, *N*B-DNJ was added freely to the culture medium, so that final concentrations ranged between 0 and 750 nM. In separate incubations, *N*B-DNJ DCPP liposomes were added so that the final lipid concentration was 50 µM and final concentrations of *N*B-DNJ ranged between 0 and 750 nM. Experiments were carried out in duplicate in 6-well plates. The quantity of secreted BVDV viral particles was measured following 3 days of incubation. Virus secretion analysis was performed by quantitative PCR (real-time PCR) on viral RNA extracted from 500 µl of supernatant. Real-time PCR used primers directed against the ncp BVDV RNA where the forward primer sequence was TAGGGCAAACCATCTGGAAG, and the reverse primer sequence was ACTTGGAGCTACAGGCCTCA. Results are expressed as the percentage of RNA copies present in treated samples as compared to the untreated control (= 100% on the x axis).

Figure 5 represents results on the effects of *N*B-DNJ, both free and in DCPP liposomes, on the secretion of ncp BVDV. Using a final concentration of 750 nM *N*B-DNJ, there is a 2-fold decrease in the number of secreted viral particles with liposome-mediated delivery, and no effect with free delivery. Assays can be further optimized to decrease viral secretion even further through increasing either liposome or encapsulated *N*B-DNJ concentrations.

Although the present invention is not limited by its theory of operation, the mechanism, by which BVDV secretion could be inhibited, may be through the retention of viral glycoproteins, such as E1 and E2, within the ER as a result of glucosidase inhibition.

### BVDV INFECTIVITY

In the following example, DCPP liposomes, both empty and encapsulating *N*B-DNJ, are shown to decrease the infectivity of BVDV particles secreted from MDBK cells. DCPP liposomes and *N*B-DNJ are also shown to work synergistically.

Using BVDV viral particles collected from the previous experiment (500 µl of supernatant containing secreted BVDV), the infectivity of secreted *N*B-DNJ-treated BVDV was determined by incubation with naive MDBK cells for 3 days followed by immunofluorescent staining to identify BVDV proteins in infected cells. Cells were stained post-infection using an anti-BVDV NS2-NS3 monoclonal antibody, followed by a FITC-labelled secondary antibody. Experiments were performed in duplicate using 6-well plates. The percent viral infectivity was calculated by the number of infected cells, identified by the presence of non-structural BVDV proteins, divided by the total cell count determined by DAPI staining of cell nuclei. Results were also normalized to account for decreased or increased viral titres in each sample (results from BVDV secretion experiments).

Figure 6 represents results on the effects of *N*B-DNJ, both free and in DCPP liposomes, on the infectivity of ncp BVDV. These results demonstrate that untreated BVDV, as well as BVDV treated with free *N*B-DNJ up to a final concentration of 750 nM, produced viral progeny that were able to infect approximately 20 % of naive MDBK cells. Infected cells incubated with 750-nM *N*B-DNJ delivered via DCPP liposomes, however, significantly reduced infectivity of viral progeny (less than 1 % of naive cells were infected). Surprisingly, all infected MDBK cells treated with DCPP liposomes, either with or without encapsulated *N*B-DNJ, reduced the number of cells infected by viral progeny to approximately 7 %. Therefore, DCPP liposomes encapsulating only 1 x PBS reduced BVDV infectivity almost 3-fold compared to untreated virus, however, in combination with 750-nM *N*B-DNJ antiviral effects were increased to over 20-fold greater than controls. *N*B-DNJ added freely to the medium at the same concentration, 750 nM, had little to no effect. Assays can be further optimized to completely abolish viral infectivity through increasing either liposome or encapsulated *N*B-DNJ concentrations.

Although the invention is not limited by its principle of operation, the mechanism, by which viral infectivity is affected by treatment with *N*B-DNJ encapsulating DCPP liposomes, can be a combined effect of DCPP lipids integrating into the ER membrane, as well as a targeted delivery of the antiviral agent, *N*B-DNJ, directly to its site of action leading to enhanced activity and misfolding of viral glycoproteins. As a result, viral progeny treated with these liposomes can have DCPP lipids present in their viral envelope, in addition to viral glycoproteins that are defective due to the lack of glycan processing in the ER.

### ANTIVIRAL EFFECT OF LIPOSOME ENCAPSULATED NB-DNJ

The antiviral effect of liposome-included NB-DNJ was also tested against the cp BVDV, using the yield reduction assay, a method which takes advantage of the cp strain being able to form plaques on mono layers of infected cells.

Figure 7 represents the effect of free vs. DC liposome-encapsulated NB-DNJ, on secretion of infectious cp BVDV. MDBK cells infected with cp BVDV were treated for 3 days with either free or DC liposome-*N*B-DNJ, so that the final *N*B-DNJ concentrations ranged between 0 and 500 µM and the total lipid concentration was 100 µM. The supernatants were then removed and used to infect fresh MDBK monolayers in six-well plates. After 3 days the plaques were counted under the microscope (plaque assay) and the results were expressed as a percentage of the number of plaques resulting from infection with no drug supernatant (=100%) (x axis). The y axis indicates the free or DC liposome-included NB-DNJ concentrations used in the plaque assay. The IC50 is indicated at the bottom of the graph. The results show that *N*B-DNJ inclusion into the DC liposomes resulted in a 8 fold better inhibition of secretion of infectious BVDV (IC50 of 20 µM, compared to 175 µM for the free drug). The assay on the cp BVDV can be optimized by incorporating *N*B-DNJ into DCPP liposomes, which can be more stable in the cell culture medium and hence have an improved antiviral effect.

### INCREASED ACTIVITY OF DCPP ENCAPSULATED NB-DNJ MEASURED BY FREE OLIGOSACCHARIDE ANALYSIS

Free oligosaccharides (FOS) produced in the presence of free or DCPP liposomes encapsulating *N*B-DNJ are characterized and used as a cellular marker for glucosidase inhibition to measure the enhancement of drug activity due to intracellular delivery. FOS have been shown to be generated through the action of a cytosolic peptide:N-glycanase (PNGase) on misfolded glycoproteins exported from the ER for proteasomal degradation via the Sec61-containing channel in the ER membrane. The identification of glycans present in FOS reveals at what stage protein folding was interrupted. In this experiment, the distribution of glucosylated FOS is used as a measure of glucosidase inhibition by *N*B-DNJ, where Glc₁Manₙ-FOS and Glc₂Mann-FOS species are a result of glucosidase II inhibition, and Glc₃Manₙ-FOS a result of glucosidase I inhibition.

CHO cells were incubated in the presence of free *N*B-DNJ at the antiviral concentration of 0.5 mM, or with 100 µM of DCPP liposomes encapsulating *N*B-DNJ at final concentrations ranging 0 - 75 nM. Cells were left to incubate for 5 days and isolation of FOS was performed using cell homogenates standardized to 500 µg of total protein as described by Mellor et al (2004). Biochem J. 2004 August 1; 381(Pt 3): 861-866. Detection of FOS was performed by normal-phase HPLC following labeling of oligosaccharides by 2-aminobenzamide. The compositions of FOS were further characterized and determined by digestions with glycosidases including endoglycosidase H, jack bean α-mannosidase, and α-glucosidases I and II. The percentage of non-glucosylated and glucosylated oligomannose FOS (represented by Manₙ-FOS, Glc₁Manₙ-FOS, Glc₂Manₙ-FOS, and Glc₃Manₙ-FOS) was calculated for each sample, and results are represented in Table 2. Results represent the mean ± S.D. of four separate experiments.

**Table 2. Distribution of FOS produced in the presence of free or DCPP liposome-delivered NB-DNJ in CHO cells.**

| Distribution of total FOS (%±S.D.) | | | | |
|---|---|---|---|---|
| *N*B-DNJ Sample | Glc₀Manₙ | Glc₁Manₙ, | Glc₂Manₙ | Glc₃Manₙ |
| untreated | 78.8±4.3 | 16.0±2.4 | 3.4±0.5 | 1.8±0.2 |
| 0.5 mM free | 11.9±**1.9** | 9.5±0.9 | 31.7±4.5 | 46.9±4.8 |
| PBS in liposomes | 81.2±6.4 | 15.2±4.8 | 2.9±1.8 | 0.7±0.5 |
| 0.75 pM in liposomes | 70.7±5.4 | 18.7±3.7 | 7.7±1.1 | 2.9±0.9 |
| 7.5 pM in liposomes | 70.4±4.2 | 17.7±1.3 | 8.3±2.1 | 3.6+1.3 |
| 75 pM in liposomes | 51.7±4.7 | 38.6±4.1 | 5.3±1.8 | 4.4±2.8 |
| 0.75 nM in liposomes | 20.4±2.7 | 66.9±5.1 | 7.4±3.3 | 5.3±2.9 |
| 7.5 nM in liposomes | 12.7±2.6 | 50.0±4.8 | 28.6±2.7 | 8.7±1.7 |
| 75 nM in liposomes | 13.2±4.1 | 7.9±2.8 | 26.6±4.5 | 52.3±6.2 |

CHO cells incubated in the presence of free *N*B-DNJ at the antiviral concentration of 0.5 mM produced primarily triglucosylated species of FOS, indicating inhibition of glucosidase I at this concentration. This can suggest that inhibition of glucosidase I is responsible for the antiviral effects of *N*B-DNJ previously reported for HIV. FOS isolated from liposome-treated samples with increasing *N*B-DNJ concentrations revealed that the distribution of glucosylated FOS gradually shifted from primarily Glc₁Man-FOS to Glc₃Man-FOS, with the inhibition of glucosidase II and I, respectively. When delivered via DCPP liposomes, samples treated with a final concentration of 750 nM *N*B-DNJ demonstrated comparable levels of inhibition of glucosidase I as seen for free incubations at 0.5 mM, suggesting an approximate 600 to 700-fold enhancement of antiviral activity as a result of intracellular delivery.

Although the invention is not bound by its principle of operation, the mechanism, by which increased *N*B-DNJ activity is achieved, can be through the direct delivery of this antiviral agent to its site of action (i.e. the ER lumen). Direct ER delivery can allow the agent to bypass both the plasma and ER membranes, which potentially can act as barriers when *N*B-DNJ is added freely to the surrounding medium. The increased levels of intracellular delivery observed when *N*B-DNJ is used for encapsulation may also contribute to an enhancement of activity as a result of higher concentrations of *N*B-DNJ within the ER lumen compared to other compounds.

### INCREASED ACTIVITY OF DCPP ENCAPSULATED NB-DNJ DETERMINED BY 2G12 ANTIBODY BINDING

In the following example, the activity of *N*B-DNJ as measured by the inhibition of glycan processing on the HIV envelope protein, gp120, is determined following expression in CHO cells in the presence of free or DCPP liposome-encapsulated *N*B-DNJ.

CHO cells expressing a soluble form of HIV gp120 were incubated in the presence of free *N*B-DNJ with concentrations ranging between 0 - 5 mM, or with liposomes encapsulating *N*B-DNJ with final concentrations in the medium ranging between 0 and 750 nM. Cells were left to incubate for 5 days before cellular supernatant containing the treated gp120 was collected. To measure the inhibition of glycan processing on gp120 expressed in the presence of *N*B-DNJ (either free or in liposomes), the binding of the MAb 2G12 was determined by capture ELISA. 2G12 recognizes a cluster of mannose residues on the carbohydrate-rich surface of gp120, and loss of binding in the presence of *N*B-DNJ results from the retention of glucose on the oligomannose glycans that form the epitope. A loss of binding affinity, however, may also arise from a misfolding of the protein, and to distinguish between these two possibilities, the affinity of all samples for the neutralizing MAb, b12, is also measured. The b12 antibody recognizes a conformationally-sensitive epitope, the CD4 binding site, which does not overlap with that of 2G12. Soluble gp120 located in the cellular supernatant was captured in ELISA plates using the D7324 antibody (binds the C5 region of gp120) and treated with 10 µg/ml of either 2G12 or b12. The binding of both antibodies to *N*B-DNJ-treated samples was related to that for the untreated gp120, where data are expressed as percent binding and represent the mean ± S.D. of triplicates from four separate experiments.

Figure 8 demonstrates a loss of gp120 binding to 2G12 (1.2 ± 0.1 % binding) following treatment with 0.5-mM *N*B-DNJ free in the medium, while maintaining 100 % binding to b 12. Liposome-mediated delivery with a final concentration of 7.5 nM *N*B-DNJ resulted in 9.8 ± 4.8 % binding to 2G12 with no significant effect on b 12 binding (96.3±3.2 %), demonstrating a 60,000 to 70,000-fold enhancement of the IC90 as a result of intracellular delivery.

### LIPOSOME DELIVERY OF IMMUNOPOTENTIATING PEPTIDES

The inventors have also discovered that one can increase presentation by major histocompatibility molecule class 1 by contacting an antigen presenting cell with a composition that contains a pH sensitive liposome, such as a liposome comprising DOPE, CHEMS and/or PEG-PE lipids, and an antigen, such as an immunopotentiating peptide, encapsulated in the liposome. Such contacting can be a result of administering the composition to a subject that comprises the antigen presented cell. The administration of the composition to the subject can be used for vaccinating the subj ect.

The immunopotenting peptide can be exemplified by a tyrosinase peptide, YMDGTMSQV, which has been found to be presented by a major histocompatibility molecule 1, HLA-A0201, on cells expressing full-length tyrosinase. This is a converted peptide resulted from the tyrosinase peptide, YMNGTMSQV corresponding to tyrosinase amino acids 369-377 and including the N-linked glycosylation site 6. Although the present invention is not limited by its theory of operation, the converted peptide probably can arise as a result of the deglycosylation in the cytosol by the enzyme peptide: N-glycanase. N-glycanase peptide binds to the transporter associated with antigenic processing (TAP), which transports the peptide into the ER. The encapsulation of the YMDGTMSQV peptide into DCPP liposomes reduces the ER delivery of the peptide by an order of magnitude.

### TREATMENT OF HIV-1-INFECTED PBMCs WITH FREE AND LIPOSOME-ENCAPSULATED NB-DNJ

Clearance of HIV-1 primary isolates from infected human cells by *N*B-DNJ was assessed using phytohemagglutinin (PHA)-activated peripheral blood mononuclear cells (PBMCs) as indicator cells and the determination of p24 antigen production as the end point.

PBMCs from four normal (uninfected) donors were isolated, pooled, and stimulated with PHA (5 µg/ml) for 48 h followed by PHA plus interleukin-2 (40 U/ml) for 72 h in RPMI 1640 medium containing 10% heat-inactivated fetal bovine serum (FBS), 100 U of penicillin per ml, 100 µg of streptomycin per ml, and 2 mM L-glutamine. All experiments were performed in 96-well microtiter plates. To infect cells, 100 µl of PHA-activated PBMCs (5x10⁵/ml) was added to each well, after which an equal volume containing 100 50% tissue culture infective doses (TCID50) of primary isolate stock was added. After an overnight incubation, the cells were washed three times with tissue culture medium, and finally re-suspended in medium containing the appropriate liposome treatment or free *N*B-DNJ. On day 7, approximately 10 - 30 % of the culture volume containing secreted HIV-1 virions was used to infect naive PBMCs for a second round of infection and treatment (volume transferred was calculated to be the volume necessary for the untreated control of that isolate to infect naive cells at a TCID50 = 100). Rounds of treatment and infection of naive cells were continued over four weeks, and at every time point cellular supernatant containing secreted virions was isolated and used in capture ELISAs to determine p24 concentration (measure of secretion). Additionally, supernatant isolated at each time point throughout treatment was used to infect naive PBMCs (TCID50 = 100) for two weeks with no further treatment, which allowed for the observation of a rebound in viral activity.

PBMCs infected with 8 primary isolates (listed in Table 3) were incubated in the presence of free *N*B-DNJ (concentrations ranging 0 - 1 mM) or liposome-encapsulated *N*B-DNJ (0 - 3.75 µM, final lipid concentration of 50 µM).

**Table 3. List of HIV-1 primary isolates used in assays, including clade identification and tropism.**

| Primary isolate | Clade | Tropism | Primary isolate | Clade | Tropism |
|---|---|---|---|---|---|
| 92UG037 | A | R5 | 93IN101 | C | R5 |
| 92RW021 | A | R5 | 97USNG30 | C | R5 |
| JR-FL | B | R5 | 92UG021 | D | X4 |
| 92HT599 | B | X4 | 92UG046 | D | X4 |
| 89.6 | B | R5/X4 | 93BR020 | F | R5/X4 |

Results from PBMCs treated with free *N*B-DNJ confirmed the antiviral concentration against HIV-1 as being 500 µM. This was the lowest concentration to clear viral activity over four weeks treatment in all isolates (Figure 9a). All isolates demonstrated at least a 90 % reduction in viral activity (IC90) suggesting that *N*B-DNJ is able to target a broad range of HIV-1 effectively.

The effect of free *N*B-DNJ on viral secretion can be determined from p24 measurements taken following the first week of treatment. At the highest concentration of *N*B-DNJ, 1 mM free in medium, most isolates responded with an approximate 30 - 40 % reduction in viral secretion (Figure 9b). Of particular interest are the three clade b isolates, 89.6, JR-FL, and 92HT599, where secretion was reduced by 50 %, and in the case of 89.6, 75 %. 93IN101, a clade c isolate, also had a 50 % decrease in secretion.

The extent of *N*B-DNJ's antiviral activity on the different primary isolates (drug sensitivity) was estimated from p24 measurements taken following three rounds of treatment. At this point a full curve of p24 secretion was observed for each isolate, and therefore both the IC50 and IC90 could be calculated. Data for the eight isolates tested are shown in Figure 9c, where six isolates are calculated to have an IC50 and IC90 of 400 µM and 500 µM, respectively, and two isolates, 250 µM and 375 µM, respectively. The two isolates shown to be the most sensitive to µB-DNJ treatment, 89.6 and 93BR3020, are also the only isolates included in these experiments known to exhibit dual tropism, whereas the other six isolates are either R5 or X4 (mono) tropic.

The antiviral activity of *N*B-DNJ, when encapsulated within DOPE:CHEMS:PEG-PE liposomes, was measured in the same eight isolates to determine enhanced activity with intracellular delivery. Figure 10 shows the level of p24 secretion over four weeks treatment in the eight different isolates tested (graphs A-H) with final concentrations of *N*B-DNJ ranging 0 - 3.75 µM. When virus reduction is compared to results using 500 µM free *N*B-DNJ all isolates demonstrate similar patterns of antiviral activity with final *N*B-DNJ concentrations ranging 3.75 - 37.5 nM, an enhancement of approximately 10⁴- 10⁵-fold. Again, there was variation in the sensitivity of the different isolates to treatment with *N*B-DNJ liposomes as new variables exist such as rate of liposome uptake and efficiency of intracellular delivery in addition to drug sensitivity.

### TARGETING HIV-1 INFECTED PBMCs WITH sCD4-LIPOSOMES AND IMMUNOLIPOSOMES

Increased cellular uptake by targeting liposomes to the gp120/gp41 complex expressed on the surface of HIV-1-infected cells was assessed in nine different primary isolates using a soluble CD4 molecule (sCD4) and several monoclonal antibodies known to bind this complex.

sCD4-liposome conjugates were created by first chemically reacting the primary amine of sCD4 with *N*-succinimidyl-S-acetylthiopropionate to create a protected sulfhydryl group, which was then unprotected by deacetylation with hydroxylamine•HCl. Immunoliposomes were prepared by first reducing IgG molecules with 2-mercaptoethanolamine, an agent that specifically reduces the disulfide bonds in the hinge region between the two heavy chains, creating two half IgG molecules each containing free sulfliydryls. Liposomes were prepared as previously described, however a PE lipid containing a maleimide group (MCC-PE) was incorporated into the bilayer so that the final liposome composition was DOPE:CHEMS:PEG-PE:MCC-PE:Rh-PE (molar ratio 6:4:0.3:0.3:0.1). Unprotected sCD4 molecules and reduced IgG molecules were left to incubate with liposomes overnight at room temperature. Liposomes were purified from free sCD4 or IgG using size exclusion chromatography.

Fluorescent-labeled lipids (Rh-PE) were incorporated into the liposome bilayer, and increased endocytosis was calculated from the increase in fluorescence detected in cells following incubation. PBMCs were purified, cultured, and infected in 96-well microtiter plates as previously described. PBMCs were left to incubate with primary isolates (TCIC50 = 100) five days before cells were washed three times with tissue culture medium, and finally resuspended in medium containing the appropriate liposome treatment (final lipid concentration of 50 µM). Following a 24 h incubation, PBMCs were isolated, washed twice with 200 µ1 PBS, and resuspended in 50 µ1 PBS with 1% (vol/vol) Empigen. Fluorescence was measured at λₑₓ = 520 nm and λₑₘ = 590 nm.

Six separate monoclonal antibodies, IgGs b6, b12, 2G12, 2F5, X5, and 4E10, were included in the study, however b6 and 4E10 could not be conjugated to liposomes as they both caused aggregation of lipids.

Figure 11 represents results obtained using sCD4-liposomes and b12-, 2G12-2F5-, and X5-immunoliposomes encapsulating 1 mM *N*B-DNJ expressed as the percentage of liposome uptake in relation to the control. For each infection, there are significant differences in uptake between targeting molecules and the liposome only control (P<0.0001). Liposomes coupled to sCD4 were able to target all nine isolates tested and led to a significant increase in cellular uptake in relation to the liposome only control. 2F5- and b12-immunoliposomes were able to increase liposome uptake in PBMCs infected with 5 and 6 different primary isolates, respectively. 2G12- and X5-immunoliposomes did not target any of the primary isolates tested, and none of the targeting molecules caused significant liposome uptake by non-specific interactions.

Therefore, sCD4-liposomes may be the best molecule for targeting liposomes to HIV-infected cells. Not only does sCD4 successfully target a broad range of HIV-1 primary isolates, it allows for the increased uptake of liposomes in infected cells via receptor-mediated endocytosis.

### TREATMENT OF HIV-1 PBMCs WITH sCD4-LIPOSOME-ENCAPSULATED NB-DNJ

Since sCD4-liposomes were shown to have the broadest targeting ability, this liposome preparation was used in p24 secretion assays to compare the antiviral activity of *N*B-DNJ to that seen with naked liposomes or free delivery. Assays including the same group of eight primary isolates were performed with final *N*B-DNJ concentrations ranging 0 - 375 nM. Results are presented in Figure 12, where sCD4-liposomes are shown to provide an additional neutralizing capability, so that all sCD4-liposome treatments, even those containing no *N*B-DNJ, completely neutralized each primary isolate (graphs A-H).

### REBOUND OF HIV-1 VIRAL ACTIVITY

Table 4 summarizes data representing the rebound of viral activity following removal of all *N*B-DNJ treatments. In all cases, once viral activity was reduced to zero (or close to zero), there was no rebound once treatments were removed for two weeks.

**Table 4. All average p24 secretion data from HIV-1-infected PBMCs over four weeks treatment with NB-DNJ delivered either free in medium, in liposomes, or in sCD4-liposomes. To measure rebound (-rb) of viral activity, treatments are removed for two weeks before p24 secretion is measured.**

| | **% p24 secretion** | | | | | | | | |
|---|---|---|---|---|---|---|---|---|---|
| **Treatment** | ***N*B-DNJ (uM)** | **Week 1** | **Week 1-rb** | **Week 2** | **Week 2-rb** | **Week 3** | **Week 3-rb** | **Week 4** | **Week 4-rb** |
| | | | | | | | | | |

| **HIV-1 isolate: UG92021** | | | | | | | | | |
|---|---|---|---|---|---|---|---|---|---|
| | | | | | | | | | |
| Free | 0 | 100 | 100 | 100 | 100 | 100 | 100 | 100 | 100 |
| Free | 250 | 90 | 110 | 83 | 101 | 88 | 91 | 77 | 85 |
| Free | 500 | 85 | 98 | 56 | 62 | 23 | 27 | 8 | 19 |
| Free | 1000 | 61 | 72 | 21 | 19 | 5 | 3 | 0 | 0 |
| | | | | | | | | | |
| Liposome | 0 | 92 | 97 | 85 | 97 | 82 | 91 | 81 | 95 |
| Liposome | 0.000375 | 79 | 101 | 76 | 69 | 38 | 41 | 35 | 44 |
| Liposome | 0.00375 | 61 | 80 | 43 | 57 | 11 | 22 | 2 | 9 |
| Liposome | 0.0375 | 44 | 55 | 31 | 45 | 5 | 12 | 2 | 5 |
| Liposome | 0.375 | 40 | 47 | 5 | 1 | 0 | 1 | 0 | 0 |
| Liposome | 3.75 | 26 | 41 | 0 | 0 | 0 | 0 | 0 | 1 |
| | | | | | | | | | |
| sCD4-liposome | 0 | 76 | 15 | 2 | 0 | 0 | 1 | 0 | 0 |
| sCD4-liposome | 0.000375 | 59 | 22 | 3 | 2 | 1 | 0 | 0 | 0 |
| sCD4-liposome | 0.00375 | 17 | 13 | 1 | 0 | 0 | 1 | 0 | 0 |
| sCD4-liposome | 0.0375 | 24 | 11 | 0 | 0 | 0 | 0 | 0 | 0 |
| sCD4-liposome | 0.375 | 18 | 16 | 0 | 0 | 0 | 0 | 0 | 0 |
| | | | | | | | | | |

| **HIV-1 isolate: UG92046** | | | | | | | | | |
|---|---|---|---|---|---|---|---|---|---|
| | | | | | | | | | |
| Free | 0 | 100 | 100 | 100 | 100 | 100 | 100 | 100 | 100 |
| Free | 250 | 91 | 99 | 115 | 92 | 91 | 94 | 79 | 89 |
| Free | 500 | 79 | 75 | 39 | 43 | 10 | 15 | 0 | 0 |
| Free | 1000 | 64 | 64 | 15 | 21 | 3 | 1 | 0 | 0 |
| | | | | | | | | | |
| Liposome | 0 | 103 | 104 | 95 | 103 | 102 | 99 | 97 | 96 |
| Liposome | 0.000375 | 78 | 97 | 77 | 82 | 62 | 78 | 59 | 67 |
| Liposome | 0.00375 | 58 | 82 | 57 | 31 | 11 | 23 | 8 | 5 |
| Liposome | 0.0375 | 40 | 64 | 34 | 36 | 7 | 11 | 1 | 1 |
| Liposome | 0.375 | 24 | 27 | 1 | 4 | 0 | 0 | 0 | 0 |
| Liposome | 3.75 | 22 | 9 | 0 | 1 | 0 | 0 | 0 | 0 |
| | | | | | | | | | |
| sCD4-liposome | 0 | 74 | 29 | 5 | 3 | 1 | 0 | 0 | 0 |
| sCD4-liposome | 0.000375 | 43 | 22 | 4 | 0 | 0 | 0 | 0 | 0 |
| sCD4-liposome | 0.00375 | 16 | 12 | 1 | 1 | 0 | 0 | 0 | 0 |
| sCD4-liposome | 0.0375 | 21 | 14 | 0 | 0 | 0 | 1 | 0 | 0 |
| sCD4-liposome | 0.375 | 15 | 15 | 0 | 0 | 0 | 0 | 0 | 0 |
| | | | | | | | | | |

| **HIV-1 isolate: HT92599** | | | | | | | | | |
|---|---|---|---|---|---|---|---|---|---|
| | | | | | | | | | |
| Free | 0 | 100 | 100 | 100 | 100 | 100 | 100 | 100 | 100 |
| Free | 250 | 99 | 93 | 84 | 99 | 88 | 95 | 81 | 88 |
| Free | 500 | 53 | 67 | 19 | 27 | 8 | 17 | 1 | 1 |
| Free | 1000 | 55 | 52 | 10 | 15 | 3 | 6 | 0 | 1 |
| | | | | | | | | | |
| Liposome | 0 | 91 | 100 | 82 | 98 | 95 | 102 | 88 | 95 |
| Liposome | 0.000375 | 68 | 94 | 64 | 104 | 66 | 72 | 39 | 68 |
| Liposome | 0.00375 | 54 | 68 | 34 | 18 | 11 | 21 | 1 | 7 |
| Liposome | 0.0375 | 45 | 74 | 19 | 22 | 8 | 11 | 1 | 2 |
| Liposome | 0.375 | 38 | 41 | 5 | 2 | 1 | 1 | 0 | 0 |
| Liposome | 3.75 | 33 | 11 | 1 | 7 | 1 | 0 | 0 | 0 |
| | | | | | | | | | |
| sCD4-liposome | 0 | 63 | 10 | 0 | 0 | 1 | 1 | 0 | 0 |
| sCD4-liposome | 0.000375 | 40 | 12 | 0 | 0 | 0 | 0 | 0 | 0 |
| sCD4-liposome | 0.00375 | 23 | 2 | 0 | 0 | 0 | 1 | 0 | 1 |
| sCD4-liposome | 0.0375 | 15 | 1 | 0 | 0 | 0 | 0 | 0 | 1 |
| sCD4-liposome | 0.375 | 16 | 2 | 0 | 0 | 0 | 0 | 0 | 0 |
| | | | | | | | | | |

| **HIV-1 isolate: BR93020** | | | | | | | | | |
|---|---|---|---|---|---|---|---|---|---|
| | | | | | | | | | |
| Free | 0 | 100 | 100 | 100 | 100 | 100 | 100 | 100 | 100 |
| Free | 250 | 89 | 97 | 74 | 77 | 53 | 71 | 55 | 59 |
| Free | 500 | 60 | 68 | 19 | 3 | 1 | 0 | 1 | 1 |
| Free | 1000 | 58 | 44 | 2 | 0 | 0 | 0 | 0 | 0 |
| | | | | | | | | | |
| Liposome | 0 | 102 | 99 | 99 | 89 | 88 | 92 | 91 | 93 |
| Liposome | 0.000375 | 91 | 105 | 75 | 59 | 55 | 59 | 57 | 46 |
| Liposome | 0.00375 | 56 | 88 | 28 | 37 | 19 | 21 | 15 | 9 |
| Liposome | 0.0375 | 48 | 57 | 21 | 12 | 4 | 5 | 3 | 0 |
| Liposome | 0.375 | 50 | 39 | 7 | 4 | 1 | 0 | 1 | 0 |
| Liposome | 3.75 | 43 | 17 | 1 | 0 | 0 | 1 | 0 | 0 |
| | | | | | | | | | |
| sCD4-liposome | 0 | 93 | 56 | 13 | 7 | 2 | 0 | 0 | 0 |
| sCD4-liposome | 0.000375 | 70 | 41 | 9 | 4 | 0 | 0 | 1 | 0 |
| sCD4-liposome | 0.00375 | 64 | 27 | 6 | 5 | 0 | 0 | 1 | 0 |
| sCD4-liposome | 0.0375 | 37 | 13 | 2 | 0 | 0 | 0 | 0 | 0 |
| sCD4-liposome | 0.375 | 31 | 9 | 1 | 0 | 0 | 0 | 0 | 0 |
| | | | | | | | | | |

| **HIV-1 isolate: 89.6** | | | | | | | | | |
|---|---|---|---|---|---|---|---|---|---|
| | | | | | | | | | |
| Free | 0 | 100 | 100 | 100 | 100 | 100 | 100 | 100 | 100 |
| Free | 250 | 98 | 81 | 79 | 70 | 54 | 73 | 59 | 66 |
| Free | 500 | 36 | 35 | 16 | 8 | 2 | 0 | 0 | 0 |
| Free | 1000 | 25 | 21 | 1 | 0 | 1 | 0 | 0 | 0 |
| | | | | | | | | | |
| Liposome | 0 | 93 | 89 | 93 | 92 | 91 | 101 | 88 | 92 |
| Liposome | 0.000375 | 85 | 83 | 47 | 33 | 42 | 57 | 31 | 27 |
| Liposome | 0.00375 | 35 | 55 | 12 | 12 | 1 | 0 | 2 | 1 |
| Liposome | 0.0375 | 24 | 32 | 4 | 2 | 0 | 0 | 0 | 0 |
| Liposome | 0.375 | 17 | 10 | 0 | 0 | 0 | 0 | 1 | 0 |
| Liposome | 3.75 | 8 | 4 | 0 | 0 | 0 | 0 | 0 | 0 |
| | | | | | | | | | |
| sCD4-liposome | 0 | 54 | 52 | 2 | 2 | 0 | 0 | 1 | 0 |
| sCD4-liposome | 0.000375 | 13 | 14 | 0 | 0 | 1 | 0 | 0 | 0 |
| sCD4-liposome | 0.00375 | 13 | 2 | 0 | 0 | 0 | 0 | 0 | 0 |
| sCD4-liposome | 0.0375 | 7 | 1 | 0 | 0 | 0 | 0 | 0 | 0 |
| sCD4-liposome | 0.375 | 4 | 2 | 0 | 0 | 0 | 0 | 0 | 0 |
| | | | | | | | | | |

| **HIV-1 isolate: JR-FL** | | | | | | | | | |
|---|---|---|---|---|---|---|---|---|---|
| | | | | | | | | | |
| Free | 0 | 100 | 100 | 100 | 100 | 100 | 100 | 100 | 100 |
| Free | 250 | 107 | 104 | 105 | 101 | 89 | 97 | 93 | 91 |
| Free | 500 | 64 | 75 | 24 | 9 | 5 | 4 | 0 | 2 |
| Free | 1000 | 44 | 56 | 9 | 5 | 1 | 1 | 0 | 0 |
| | | | | | | | | | |
| Liposome | 0 | 92 | 100 | 99 | 97 | 104 | 98 | 96 | 99 |
| Liposome | 0.000375 | 94 | 111 | 98 | 95 | 87 | 93 | 79 | 90 |
| Liposome | 0.00375 | 72 | 77 | 39 | 11 | 11 | 19 | 13 | 19 |
| Liposome | 0.0375 | 50 | 34 | 16 | 14 | 8 | 7 | 5 | 3 |
| Liposome | 0.375 | 32 | 16 | 5 | 7 | 1 | 2 | 1 | 0 |
| Liposome | 3.75 | 32 | 6 | 0 | 0 | 0 | 0 | 0 | 0 |
| | | | | | | | | | |
| sCD4-liposome | 0 | 56 | 24 | 4 | 5 | 1 | 0 | 1 | 0 |
| sCD4-liposome | 0.000375 | 35 | 17 | 1 | 0 | 0 | 0 | 0 | 0 |
| sCD4-liposome | 0.00375 | 17 | 1 | 0 | 0 | 0 | 0 | 0 | 0 |
| sCD4-liposome | 0.0375 | 14 | 0 | 0 | 0 | 0 | 0 | 0 | 0 |
| sCD4-liposome | 0.375 | 21 | 0 | 0 | 0 | 0 | 0 | 0 | 0 |
| | | | | | | | | | |

| **HIV-1 isolate: 93IN101** | | | | | | | | | |
|---|---|---|---|---|---|---|---|---|---|
| | | | | | | | | | |
| Free | 0 | 100 | 100 | 100 | 100 | 100 | 100 | 100 | 100 |
| Free | 250 | 106 | 96 | 81 | 88 | 78 | 81 | 66 | 72 |
| Free | 500 | 75 | 88 | 45 | 37 | 7 | 11 | 2 | 0 |
| Free | 1000 | 67 | 51 | 17 | 4 | 3 | 1 | 0 | 0 |
| | | | | | | | | | |
| Liposome | 0 | 109 | 102 | 101 | 105 | 96 | 101 | 92 | 95 |
| Liposome | 0.000375 | 96 | 109 | 97 | 101 | 44 | 67 | 36 | 41 |
| Liposome | 0.00375 | 76 | 99 | 53 | 72 | 14 | 18 | 6 | 4 |
| Liposome | 0.0375 | 64 | 57 | 24 | 19 | 5 | 7 | 2 | 1 |
| Liposome | 0.375 | 48 | 17 | 6 | 7 | 0 | 0 | 0 | 0 |
| Liposome | 3.75 | 48 | 20 | 4 | 1 | 1 | 0 | 0 | 0 |
| | | | | | | | | | |
| sCD4-liposome | 0 | 86 | 40 | 20 | 21 | 7 | 11 | 0 | 1 |
| sCD4-liposome | 0.000375 | 61 | 36 | 6 | 9 | 5 | 9 | 1 | 0 |
| sCD4-liposome | 0.00375 | 40 | 14 | 1 | 3 | 0 | 0 | 0 | 0 |
| sCD4-liposome | 0.0375 | 20 | 3 | 0 | 0 | 0 | 0 | 0 | 0 |
| sCD4-liposome | 0.375 | 23 | 0 | 0 | 0 | 0 | 0 | 0 | 0 |
| | | | | | | | | | |

| **HIV-1 isolate: 97USNG30** | | | | | | | | | |
|---|---|---|---|---|---|---|---|---|---|
| | | | | | | | | | |
| Free | 0 | 100 | 100 | 100 | 100 | 100 | 100 | 100 | 100 |
| Free | 250 | 96 | 112 | 102 | 109 | 100 | 97 | 95 | 101 |
| Free | 500 | 74 | 82 | 45 | 19 | 8 | 7 | 3 | 1 |
| Free | 1000 | 46 | 53 | 9 | 3 | 0 | 0 | 0 | 0 |
| | | | | | | | | | |
| Liposome | 0 | 93 | 99 | 98 | 104 | 97 | 101 | 93 | 95 |
| Liposome | 0.000375 | 83 | 93 | 80 | 79 | 77 | 81 | 62 | 77 |
| Liposome | 0.00375 | 47 | 58 | 32 | 46 | 28 | 32 | 13 | 2 |
| Liposome | 0.0375 | 48 | 36 | 11 | 9 | 9 | 11 | 6 | 3 |
| Liposome | 0.375 | 34 | 13 | 1 | 1 | 0 | 0 | 0 | 0 |
| Liposome | 3.75 | 20 | 11 | 0 | 0 | 0 | 0 | 0 | 0 |
| | | | | | | | | | |
| sCD4-liposome | 0 | 73 | 55 | 26 | 15 | 15 | 4 | 1 | 0 |
| sCD4-liposome | 0.000375 | 41 | 14 | 5 | 9 | 3 | 1 | 0 | 0 |
| sCD4-liposome | 0.00375 | 20 | 8 | 0 | 0 | 0 | 1 | 0 | 0 |
| sCD4-liposome | 0.0375 | 17 | 3 | 0 | 0 | 0 | 0 | 0 | 0 |
| sCD4-liposome | 0.375 | 23 | 4 | 1 | 0 | 0 | 0 | 0 | 0 |

### INCORPORATION OF PHOSPHATIDYLINOSITOL INTO DOPE:CHEMS LIPOSOMES

Phosphatidylinositol (PI) purified from bovine liver cells was incorporated into the previously assayed liposome composition of DOPE:CHEMS:Rh-PE at a final molar concentration of 10-30%. DOPC:CHEMS:Rh-PE liposomes were included in the study as a negative control. Liposomes were prepared as previously described. MDBK cells were grown to 50 % confluency before media was exchanged and replaced with fresh media containing liposomes with a final lipid concentration of 100 µM. After a 15 m incubation, liposomes were removed and cells were washed twice in PBS. Cells were incubated in fresh media for 0, 1, 2, 5, 24, or 48 hours before cells were fixed in 2.5% paraformaldehyde and visualized under a fluorescent microscope. Cells were stained with DAPI prior to visual analysis.

Figure 13 shows representative fluorescent images, taken following a 15 m pulse of each liposome preparation with MDBK cells, at time points 0, 1, 2, 5, 24, and 48 hours. Results demonstrate that liposomes containing PI lipids at final concentrations between 10 and 30% have increased lifetime within the cell, which can mean that lipids delivered via this method are more efficiently retained inside the cell, or alternatively, less efficiently degraded. Fluorescent images taken following DOPE:CHEMS:Rh-PE liposome incubation with MDBK cells show that these lipids are mostly degraded sometime between 5h and 24 h, and completely degraded by 48 h. After 48 h, PI containing liposomes were still very evident within the treated cells, and a more diffuse pattern of Rh-PE is observed. This result can indicate that at this time point most lipids have incorporated into cellular membranes and are no longer concentrated in vesicles (punctate fluorescent pattern).

### INCORPORATION OF DOPE:CHEMS LIPOSOMES INTO THE ENVELOPE OF ER-BUDDING VIRAL PARTICLES

To investigate whether liposomes are able to fuse with cellular membranes, such as the ER, liposomes containing Rh-PE were used to monitor the uptake into viral particles budding from the ER membrane. MDBK cells persistently infected with a cytopathic BVDV (NADL, MOI=0.01) and naive (uninfected) MDBK are incubated in the presence of DOPE:CHEMS:Rh-PE, DOPE:CHEMS:PI:Rh-PE, or DOPC:CHEMS:Rh-PE liposomes at a final lipid concentration of 50 µM for two days. Following the incubation, media containing liposomes was removed and cells were washed twice in PBS before fresh media was added and cells were incubated a further three days. After three days a sample of supernatant from each set of treated cells (both infected and uninfected) is taken and used for fluorescent measurement using a spectrofluorometer set at λₑₓ=550 nm and λₑₘ=590 nm. Experiments were carried-out in triplicate, and results represent the average of the three readings.

Any increase in fluorescence in the supernatant of infected cells compared to the uninfected cells treated with the same liposome preparation is due to the secretion of viral particles containing the Rh-PE lipid in their envelope. Figure 14 shows results obtained using three different lipid compositions, and these data indicate that liposomes containing DOPE (PE) are able to incorporate into the ER membrane, and subsequently into the budding viral envelope. Supernatant from infected cells treated with DOPE liposomes all demonstrated a significant increase in fluorescence compared to uninfected samples. DOPC (PC)-containing liposomes used as a negative control (no ER targeting) demonstrated no difference in fluorescence between infected and uninfected samples. Surprisingly, and in accordance with the data presented in Figure 13, liposomes containing 10-30% PI as part of the lipid composition were able to incorporate into the viral envelopes almost 5 times more efficiently then liposomes without. This indicates that PI is responsible for the increased uptake of liposomes into the ER membrane. This result may have implications for the treatment of viruses that bud from the ER such as BVDV, HCV and HBV.

The next step in the development of antiviral therapies using ER-targeting liposomes can be incorporation of antiviral proteins within the lipid bilayer of the liposomes for delivery into the ER membrane. Antiviral proteins can include, but not limited to, viral receptors known to bind viral envelope proteins and/or mutated forms of viral envelope proteins and/or proteins known to interfere with viral envelope interactions.

The incorporation of antiviral proteins into ER-targeting liposome preparations encapsulating antiviral drugs can provide a combination of three separate antiviral strategies that may act synergistically to reduce viral infectivity: 1- direct delivery of lipids into the ER membrane that when incorporated into viral envelope will reduce viral infectivity, 2- direct delivery of proteins into the ER membrane that will incorporate into the viral envelope of budding particles and reduce infectivity, and 3- direct delivery of antiviral agents into intracellular compartments.

Although the foregoing refers to particular preferred embodiments, it will be understood that the present invention is not so limited. It will occur to those of ordinary skill in the art that various modifications may be made to the disclosed embodiments and that such modifications are intended to be within the scope of the present invention.

All of the publications, patent applications and patents cited in this specification are incorporated herein by reference in their entirety.

## Claims

1. A composition comprising
(a) a liposome comprising PI lipids and
(b) at least one of i) at least one therapeutic agent encapsulated inside the liposome, and ii) at least one protein intercalated into a lipid bilayer of the liposome; wherein a molar concentration of the PI lipids in the liposome is from 5 to 50%.

2. The composition of claim 1, wherein the liposome further comprises DOPE and CHEMS lipids.

3. The composition of any of claims 1-2, wherein the molar concentration of the PI lipids is from 10 to 30 %.

4. The composition of any of claims 1-3 for its use in treating or preventing a viral infection.

5. The composition of claim 4, wherein the viral infection is an ER membrane budding viral infection.

6. The composition of claim 5, wherein the ER budding viral infection is HBV, HCV or BVDV infection.

7. The composition of claim 4, wherein the viral infection is a plasma membrane budding infection.

8. The composition of claim 7, wherein the plasma membrane budding infection is an HIV infection.

9. The composition of any of claims 4-8, wherein the at least one therapeutic agent comprises an antiviral agent.

10. The composition of claim 9, wherein the antiviral agent comprises an α-glucosidase inhibitor.

11. The composition of claim 10, wherein the α-glucosidase inhibitor comprises *N*B-DNJ.

12. The composition of claim 9, wherein the antiviral agent comprises an ion channel inhibitor.

13. The composition of claim 9, wherein the antiviral agent comprises an iminosugar.

14. The composition of any of claims 4-8, wherein the at least one protein is an antiviral protein.

15. The composition of claim 4, for its use in treating or preventing a viral infection in human being.
